# EUROPEAN PATENT APPLICATION

(11) **EP 4 471 121 A1**
(43) Date of publication of application: **04.12.2024**
(21) Application number: 23747060.4
(22) Date of filing: 27.01.2023
(51) Int. Cl.: C12N 1/13, C12N 15/29, C07K 14/405, C07K 14/415

(54) **ALGAE, METHOD FOR IMPROVING GROWTH OF ALGAE IN DARKNESS, METHOD FOR PRODUCING ALGAE EXHIBITING IMPROVED GROWTH IN DARKNESS, DARK ACTIVE CRYPTOCHROME, AND POLYNUCLEOTIDE ENCODING DARK ACTIVE CRYPTOCHROME**

(30) Priority: 27.01.2022 JP 2022011181
(71) Applicant: DIC Corporation, Tokyo 174-8520 (JP)
(72) Inventor: HIBI Keita, Sakura-shi, Chiba 285-8668 (JP); NAKAMURA Hiromasa, Sakura-shi, Chiba 285-8668 (JP)
(74) Representative: TBK
(86) International application number: PCT/JP2023/002568
(87) International publication number: WO 2023/145850

(57) **Abstract**

An alga having a dark active cryptochrome whose activation is maintained in the dark. Also, a method for improving growth of an alga in the dark including introducing a polynucleotide encoding a dark active cryptochrome into an alga. Also, a method for producing an alga with improved growth in the dark having an improved growth rate including introducing a polynucleotide encoding a dark active cryptochrome into an alga.

## Description

### TECHNICAL FIELD

The present invention relates to an alga, a method for improving growth of an alga in the dark, a method for producing an alga with improved growth in the dark, a dark active cryptochrome and a polynucleotide encoding a dark active cryptochrome.

The present application claims a priority of patent application No. 2022-011181 filed in Japan on January 27, 2022, and the contents thereof are incorporated herein.

### BACKGROUND ART

Cryptochromes are blue light photoreceptor proteins which are widely conserved among eukaryotes. Cryptochromes are known to be involved in photomorphogenesis and regulation of circadian rhythms or the like in plants. Cryptochromes are activated by reception of blue light and play a role in regulating the response reaction adopted to the light environment. It is reported that a photomorphogenic phenotype was exhibited also in the dark in *Arabidopsis thaliana* by substituting one amino acid in the flavin adenine dinucleotide (FAD)-binding site of Cryptochrome 1 (CRY1) (NPL 1).

Recently, attempts to produce useful substances using algae have been made. To use algae industrially, it is desirable that algae can be cultured in a large scale in the dark. Algae, however, usually grow photoautotrophically, and in many cases, algae cannot grow heterotrophically in the dark, or the heterotrophic growth in the dark is slow.

*Cyanidioschyzon merolae* is usually obligate autotrophic, but strains which grow heterotrophically are reported (NPLs 2 and 3). It is reported, however, that these strains have a low maximum reached algal density in heterotrophic cultivation and that the growth rate becomes further slow after subculture.

### CITATION LIST

### NON PATENT LITERATURE

NPL 1: Nan-Nan Gu, Yan-Chun Zhang, Hong-Quan Yang. Substitution of a conserved glycine in the PHR domain of Arabidopsis cryptochrome 1 confers a constitutive light response. Mol Plant. 2012 Jan;5(1):85-97.
NPL 2: Takashi Moriyama, Natsumi Mori, and Naoki Sato. Activation of oxidative carbon metabolism by nutritional enrichment by photosynthesis and exogenous organic compounds in the red alga Cyanidioschyzon merolae: evidence for heterotrophic growth. SpringerPlus (2015) 4:559.
NPL 3: Takashi Moriyama, Natsumi Mori, Noriko Nagata, and Naoki Sato. Selective loss of photosystem I and formation of tubular thylakoids in heterotrophka11y grown red alga Cyanidioschyzon merolae. Photosynthesis Research (2019) 140, 275-287.

### SUMMARY OF INVENTION

### TECHNICAL PROBLEM

It is desirable that algae can grow excellently also in the dark for industrial use of the algae. When the growth in the dark of algae which grow poorly in the dark can be improved, the algae can be more easily used industrially.

Therefore, a problem of the invention is to provide an alga with improved growth in the dark, a method for improving growth of an alga in the dark, a method for producing an alga with improved growth in the dark, a dark active cryptochrome and a polynucleotide encoding a dark active cryptochrome.

### SOLUTION TO PROBLEM

The invention includes the following aspects.
[1] An alga having a dark active cryptochrome whose activation is maintained in the dark.
[2] The alga described in [1] which has a higher growth rate in the dark than that of an alga of the same species without the dark active cryptochrome.
[3] The alga described in [1] or [2] which has a higher maximum reached algal density in the dark than that of an alga of the same species without the dark active cryptochrome.
[4] The alga described in any one of [1] to [3], wherein the dark active cryptochrome has an amino acid sequence having a mutation in a flavin adenine dinucleotide-binding domain compared to the amino acid sequence of a dark inactive cryptochrome which is inactivated in the dark.
[5] The alga described in [4], wherein the flavin adenine dinucleotide-binding domain of the dark inactive cryptochrome contains an amino acid sequence represented by the following formula (1),

   LXZXWXXGXXXJ (1)

   [wherein in the formula, L represents a leucine residue, W represents a tryptophan residue, G represents a glycine residue, Z represents a hydrophobic amino acid residue or a polar uncharged amino acid residue, J represents a hydrophobic amino acid residue, and X represents any amino acid residue.]
[6] The alga described in [5], wherein the dark active cryptochrome has an amino acid sequence having a mutation in the amino acid sequence represented by the formula (1) compared to the amino acid sequence of the dark inactive cryptochrome.
[7] The alga described in [6], wherein the dark active cryptochrome contains an amino acid sequence in which the glycine residue at position 8 of the amino acid sequence represented by the formula (1) has been substituted with a basic amino acid residue.
[8] The alga described in any one of [1] to [7], wherein the alga is a unicellular alga.
[9] A method for improving growth of an alga in the dark, including introducing a polynucleotide encoding a dark active cryptochrome into an alga.
[10] A method for producing an alga with improved growth in the dark, including introducing a polynucleotide encoding a dark active cryptochrome into an alga.
[11] A dark active cryptochrome whose activation is maintained in the dark, having an amino acid sequence selected from the group consisting of (a) to (c) below:
   (a) an amino acid sequence in which the glycine residue at position 8 of an amino acid sequence represented by the following formula (1) has been substituted with a basic amino acid residue in the amino acid sequence of SEQ ID NO: 12, 14, 34, 37, 40, 43, 46, 49, 52, 62, 65, 68, 71, 74 or 77,

      LXZXWXXGXXXJ (1)

      [wherein in the formula, L represents a leucine residue, W represents a tryptophan residue, G represents a glycine residue, Z represents a hydrophobic amino acid residue or a polar uncharged amino acid residue, J represents a hydrophobic amino acid residue, and X represents any amino acid residue];
   (b) an amino acid sequence having a mutation of one amino acid or a plurality of amino acids in the amino acid sequence of SEQ ID NO: 12, 14, 34, 37, 40, 43, 46, 49, 52, 62, 65, 68, 71, 74 or 77, wherein the mutation includes a mutation of substituting the glycine residue at position 8 of the amino acid sequence represented by the formula (1) with a basic amino acid residue; and
   (c) an amino acid sequence which has a sequence identity of 70% or more with the amino acid sequence of SEQ ID NO: 12, 14, 34, 37, 40, 43, 46, 49, 52, 62, 65, 68, 71, 74 or 77 and in which the glycine residue at position 8 of the amino acid sequence represented by the formula (1) has been substituted with a basic amino acid residue.
[12] A polynucleotide encoding the dark active cryptochrome described in [11].

### ADVANTAGEOUS EFFECTS OF INVENTION

According to the invention, an alga with improved growth in the dark, a method for improving growth of an alga in the dark, a method for producing an alga with improved growth in the dark, a dark active cryptochrome and a polynucleotide encoding a dark active cryptochrome are provided.

### BRIEF DESCRIPTION OF DRAWINGS

[Fig. 1] The results of an alignment of candidates of CRY of C. *melorae* and *Arabidopsis thaliana* CRY1 using Clustal Omega are shown. The amino acid sequences around the "LXZXWXXGXXXJ" motif are shown. "*" indicates an amino acid residue that matches in all the analyzed sequences; ":" indicates an amino acid residue that matches in a part of the analyzed sequences; and "." indicates an amino acid residue of a matching amino acid family among the analyzed sequences (the same applies to Figs. 5 to 8).
[Fig. 2A] The structure of a linear transformation DNA vector for introducing CYME_CMM076C (G419R) gene into *C*. *merolae* is shown.
[Fig. 2B] The structure of a linear transformation DNA vector for introducing CYME_CMQ453C (G572R) gene into *C*. *merolae* is shown.
[Fig. 3] The results of evaluation of heterotrophic growth of a CMM076C (G419R)-introduced strain and a CMQ453C (G572R)-introduced strain in the dark are shown.
[Fig. 4] The results of observation of the introduced protein expression of a CMM076C (G419R)-introduced strain and a CMQ453C (G572R)-introduced strain by Western Blot analysis are shown.
[Fig. 5] The results of an alignment of a candidate of CRY of *Haematococcus pluvialis* and *Arabidopsis thaliana* CRY1 are shown.
[Fig. 6] The results of an alignment of candidates of CRY of *Nannochloropsis gaditana* and *Arabidopsis thaliana* CRY1 are shown.
[Fig. 7] The results of an alignment of candidates of CRY of *Phaeodactylum tricornutum* and *Arabidopsis thaliana* CRY1 are shown.
[Fig. 8] The results of an alignment of candidates of CRY of *Diacronema lutheri* and *Arabidopsis thaliana* CRY1 are shown.
[Fig. 9] The results of an alignment of candidates of CRY of *Chaetoceros muelleri* and *Arabidopsis thaliana* CRY1 are shown.
[Fig. 10] The results of an alignment of candidates of CRY of *Chaetoceros gracilis* and *Arabidopsis thaliana* CRY1 are shown.

### DESCRIPTION OF EMBODIMENTS

The term "comprise" means that a component other than the subject component(s) may be contained. The term "consist of" means that no component other than the subject component(s) is contained. The term "consist essentially of" means that no component other than the subject component(s) is contained in an aspect exhibiting a special function (an aspect in which the effects of the invention are completely lost or the like). In the present description, a case described with "comprise" includes the aspect of "consist of" and the aspect of "consist essentially of".

The proteins, the peptides, the polynucleotides (DNA and RNA), the vectors, the cells and the algae may be isolated ones. The "isolated" means the natural state or a state separated from other components. An "isolated" material can be a material which does not substantially contain another component. "Does not substantially contain another component" means that the other component content of the isolated component is such a value that can be ignored. The other component content of the isolated component can be, for example, 10 mass% or less, 5 mass% or less, 4 mass% or less, 3 mass% or less, 2 mass% or less, 1 mass% or less, 0.5 mass% or less or 0.1 mass% or less. The proteins, the peptides, the polynucleotides (DNA and RNA), the vectors and the cells described in the present description can be isolated proteins, isolated peptides, isolated polynucleotides (isolated DNA and isolated RNA), isolated vectors, isolated cells and isolated algae.

The nucleotide sequences are written in the one letter codes which are generally accepted unless otherwise specified. Unless otherwise specified, the nucleotide sequences are described from the 5' end to the 3' end. The nucleotide residues are sometimes simply written with adenine, thymine, cytosine, guanine, uracil and the like or in the one letter codes thereof.

The amino acid sequences are written in the one letter codes or the three letter codes which are generally accepted unless otherwise specified. Unless otherwise specified, the amino acid sequences are described from the N-terminal to the C-terminal.

A specific amino acid residue contained in a specific amino acid sequence is sometimes shown in the one letter code of the amino acid residue and the position from the N-terminal. For example, the glycine residue at position 419 from the N-terminal in the amino acid sequence of SEQ ID NO: 16 is shown with "G419".

A substitution of a specific amino acid residue contained in a specific amino acid sequence is sometimes shown using the one letter code of the amino acid residue before the substitution, the position from the N-terminal and the one letter code of the amino acid residue after the substitution. For example, the substitution of the glycine residue at position 419 from the N-terminal to an arginine residue in the amino acid sequence of SEQ ID NO: 16 is shown with "G419R".

A "gene" indicates a polynucleotide containing at least one open reading frame encoding a specific protein. The gene can contain both an exon and an intron.

The sequence identity (or homology) of base sequences or amino acid sequences is determined as a proportion of the matching bases or amino acids based on the whole base sequences or the whole amino acid sequences excluding the gaps in an alignment obtained by aligning two base sequences or amino acid sequences in such a manner that the number of corresponding bases or amino acids becomes the highest while inserting gaps to the insertion and deletion sites. The sequence identity of base sequences or amino acid sequences can be determined using various types of homology search software known in the technical field. The sequence identity value of base sequences can be obtained, for example, by calculating based on an alignment obtained using known homology search software, BLASTN. The sequence identity value of amino acid sequences can be obtained, for example, by calculating based on an alignment obtained using known homology search software, BLASTP.

The "photoautotrophic cultivation" means cultivation in the presence of light (bright place) in the absence of organic substances.

The "heterotrophic cultivation" means cultivation in the dark in the presence of organic substances.

The "mixotrophic cultivation" means cultivation in the presence of light (bright place) in the presence of organic substances.

The "photoautotrophic growth" means growth of an alga in the presence of light in the absence of organic substances. The photoautotrophic growth may be growth of the alga in photoautotrophic cultivation.

The "heterotrophic growth" means growth of an alga in the dark in the presence of organic substances. The heterotrophic growth may be growth of the alga in heterotrophic cultivation.

The "mixotrophic growth" means growth of an alga in the presence of light in the presence of organic substances. The mixotrophic growth may be growth of the alga in mixotrophic cultivation.

The "maximum reached algal density" means the algal density at the point where the growth of the alga stops or the maximum algal density which is achieved in the process of growth of the alga in the cultivation of the alga. The maximum reached algal density may be the algal density at the point where the alga has grown and reached the stationary phase.

The "wild type" means an organism, a gene, a protein or the like which exists in nature. A wild-type alga (wild-type strain) means an alga which exists in nature. A wild-type gene means a gene which a wild-type organism has. A wild-type protein is a protein that a wild-type organism has and is expressed from a wild-type gene.

### <Alga>

A first aspect of the invention is an alga having a dark active cryptochrome whose activation is maintained in the dark.

### (Alga)

The kind of the alga in the embodiment is not particularly limited. The alga may be a red alga, a brown alga, a green alga, a diatom, a yellow-green alga, a dinoflagellate, a eustigmatophyte alga, a haptophyte alga or the like. The alga may be unicellular or multicellular, but a unicellular alga is preferable. The alga preferably forms a cell colony. The wild type of the alga preferably has a cryptochrome. The wild type of the alga is preferably unable to grow heterotrophically or preferably has low ability of growing heterotrophically. Specific examples of the "low ability of growing heterotrophically" of an alga include, for example: the growth rate in heterotrophic cultivation is slower than the growth rate in photoautotrophic cultivation; the maximum reached algal density in heterotrophic cultivation is lower than the maximum reached algal density in photoautotrophic cultivation; the growth rate after subculture in heterotrophic cultivation is slower than the growth rate after subculture in photoautotrophic cultivation; and the like.

In an embodiment, the alga is a unicellular red alga, a green alga, an eustigmatophyte alga, a diatom or a haptophyte alga.

Examples of the unicellular red alga include Cyanidiophyceae, Stylonematophyceae, Porphyridiophyceae and Rhodellophyceae. Cyanidiophyceae includes *Cyanidioschyzon, Cyanidium* and *Galdieria.* In an embodiment, the alga is *Cyanidioschyzon merolae.*

The green alga may be of Chlorophyceae. Chlorophyceae includes *Haematococcus, Chlamydomonas, Gonium, Pandorina, Volvox, Dunaliella* and the like. A specific example of the green alga is *Haematococcus pluvialis.*

The eustigmatophyte alga may be of Eustigmatophyceae. Eustigmatophyceae includes *Nannochloropsis, Microchloropsis, Monodopsis, Vischeria, Chlorobotrys, Goniochloris* and the like. A specific example of the eustigmatophyte alga is *Nannochloropsis gaditana.*

The diatom may be of Bacillariophyceae. Bacillariophyceae includes *Phaeodactylum, Synedra, Asterionella, Cyclotella, Melosira, Chaetoceros, Thalassiosira, Leptocylindrus, Nitzschia, Cylindrotheca, Eucampia, Odontella* and the like. A specific example of the diatom is *Phaeodactylum tricornutum.*

The haptophyte alga may be of Haptophyceae. Haptophyceae includes Pavlovophycidae and Prymnesiophycidae. Pavlovophycidae includes *Diacronema, Pavlova, Exanthemachrysis* and the like. A specific example of the haptophyte alga is *Diacronema lutheri.*

### (Dark Active Cryptochrome)

The "dark active cryptochrome" means a cryptochrome in which the activity is maintained in the dark. A cryptochrome is a blue light photoreceptor and is activated by reception of blue light. An activated cryptochrome directly or indirectly regulates gene expression and induces photoresponse. The activation of the dark active cryptochrome is maintained also in the dark and induces similar photoresponse to that to reception of blue light. The dark active cryptochrome has a function of improving heterotrophic growth in the dark in an alga.

Specifically, the heterotrophic growth of the alga having a dark active cryptochrome in the dark is improved compared to that of an alga of the same species without the dark active cryptochrome. More specifically, the alga having a dark active cryptochrome has a higher growth rate in the dark than that of an alga of the same species without the dark active cryptochrome. Alternatively, the alga having a dark active cryptochrome has a higher maximum reached algal density in the dark than that of an alga of the same species without the dark active cryptochrome. Alternatively, the growth rate after subculture of the alga having a dark active cryptochrome does not decrease through subculture in heterotrophic cultivation in the dark.

The "alga of the same species without the dark active cryptochrome" means an alga which is of the same taxonomic species as that of the subject alga having the dark active cryptochrome and which does not have the dark active cryptochrome. In an embodiment, the "alga of the same species without the dark active cryptochrome" has substantially the same genetic background as that of the subject alga having a dark active cryptochrome gene. "Having substantially the same genetic background" means that the genome sequences excluding the dark active cryptochrome gene (or the dark active cryptochrome gene and the marker gene) are substantially the same. When the alga has the dark inactive cryptochrome described below, "having substantially the same genetic background" may mean that the genome sequences excluding the dark active cryptochrome gene and the dark inactive cryptochrome gene (or the dark active cryptochrome gene, the dark inactive cryptochrome gene and the marker gene) are substantially the same. The substantially same genome sequences have, for example, a sequence identity of 99% or more, 99.9% or more, 99.99% or more or 99.9999% or more.

When the alga having a dark active cryptochrome is a transformant produced by introducing a dark active cryptochrome gene into an alga without the dark active cryptochrome, the "alga of the same species without the dark active cryptochrome" may be the parent strain (wild-type strain) which is the subject of the gene introduction. For example, when the alga having a dark active cryptochrome is produced by introducing a dark active cryptochrome gene into *C*. *merolae* strain NEIS-1804, the "alga of the same species without the dark active cryptochrome" may be *C*. *merolae* strain NEIS-1804.

In an embodiment, the dark active cryptochrome has a mutation in a flavin adenine dinucleotide (FAD)-binding domain compared to a dark inactive cryptochrome which is inactivated in the dark. The "dark inactive cryptochrome" may be a wild-type cryptochrome. In an embodiment, the dark inactive cryptochrome is a plant-type cryptochrome. The FAD-binding domain of the dark inactive cryptochrome can be identified through domain search using a known protein domain database. An example of the protein domain database is the Pfam provided by European Bioinformatics Institute (EMBL-EBI) or the like.

The FAD-binding domain of the dark inactive cryptochrome preferably contains an amino acid sequence represented by the following formula (1).

LXZXWXXGXXXJ (1)

[In the formula, L represents a leucine residue, W represents a tryptophan residue, G represents a glycine residue, Z represents a hydrophobic amino acid residue or a polar uncharged amino acid residue, J represents a hydrophobic amino acid residue, and X represents any amino acid residue.]

In the formula (1), Z represents a hydrophobic amino acid residue or a polar uncharged amino acid residue. The hydrophobic amino acid residue may be a leucine residue, an isoleucine residue, a phenylalanine residue, a tryptophan residue, a valine residue, a methionine residue, a cysteine residue, a tyrosine residue or an alanine residue. The hydrophobic amino acid residue of Z is preferably a leucine residue, an isoleucine residue, a phenylalanine residue, a valine residue or a methionine residue, more preferably a leucine residue, an isoleucine residue or a valine residue.

The polar uncharged amino acid residue may be a serine residue, a threonine residue, a cysteine residue, an asparagine residue or a glutamine residue. The polar uncharged amino acid residue of Z is preferably a glutamine residue.

Z is preferably a hydrophobic amino acid residue or a glutamine residue, further preferably a leucine residue, an isoleucine residue, a valine residue, a methionine residue or a glutamine residue, particularly preferably a leucine residue, an isoleucine residue, a valine residue or a glutamine residue.

In the formula (1), J represents a hydrophobic amino acid residue. The hydrophobic amino acid residue may be a leucine residue, an isoleucine residue, a phenylalanine residue, a tryptophan residue, a valine residue, a methionine residue, a cysteine residue, a tyrosine residue or an alanine residue. The hydrophobic amino acid residue of J is preferably a leucine residue, an isoleucine residue, a phenylalanine residue, a valine residue, a methionine residue or a tyrosine residue, more preferably a leucine residue, an isoleucine residue, a phenylalanine residue or a tyrosine residue.

In the formula (1), X represents any amino acid residue. When X's in the formula (1) are referred to as X¹ to X⁷ in this order from the N-terminal, the formula (1) can be represented as follows.

LX¹ZX²WX³X⁴GX⁵X⁶X⁷J (1)'

X¹ is preferably a hydrophobic amino acid residue, a basic amino acid residue or a glycine residue. The hydrophobic amino acid residue is preferably a leucine residue, a tryptophan residue or a tyrosine residue. The basic amino acid residue is preferably an arginine residue or a histidine residue. X¹ is preferably a leucine residue, a tryptophan residue, a tyrosine residue, an arginine residue, a histidine residue or a glycine residue.

X² is preferably a proline residue, a serine residue, an aspartic acid residue or an arginine residue, more preferably a proline residue, a serine residue or an aspartic acid residue, further preferably a proline residue or a serine residue, particularly preferably a proline residue.

X³ is preferably a hydrophilic amino acid residue. The hydrophilic amino acid residue may be a polar uncharged amino acid residue, a basic amino acid residue or an acidic amino acid residue. The polar uncharged amino acid residue of X³ is preferably a glutamine residue or a threonine residue, more preferably a glutamine residue. The basic amino acid residue of X³ is preferably an arginine residue or a lysine residue. The acidic amino acid residue may be an aspartic acid residue or a glutamic acid residue. The acidic amino acid residue of X³ is preferably a glutamic acid residue. X³ is preferably a glutamine residue, a threonine residue, a glutamic acid residue, an arginine residue or a lysine residue.

X⁴ is preferably a hydrophobic amino acid residue, an acidic amino acid residue or a basic amino acid residue. The hydrophobic amino acid residue of X⁴ is preferably an isoleucine residue, a tryptophan residue or an alanine residue. The acidic amino acid residue of X⁴ is preferably a glutamic acid residue or an aspartic acid residue. The basic amino acid residue of X⁴ is preferably a histidine residue or a lysine residue. X⁴ is preferably an isoleucine residue, a tryptophan residue, an alanine residue, a glutamic acid residue, an aspartic acid residue, a histidine residue or a lysine residue.

X⁵ is preferably a hydrophobic amino acid residue or an acidic amino acid residue. The hydrophobic amino acid residue of X⁵ is preferably an alanine residue, a leucine residue or a methionine residue. The acidic amino acid residue of X⁵ is preferably a glutamic acid residue. X⁵ is preferably an alanine residue, a leucine residue, a methionine residue or a glutamic acid residue.

X⁶ is preferably a basic amino acid residue, a polar uncharged amino acid residue, an acidic amino acid residue or an alanine residue. The basic amino acid residue of X⁶ is preferably an arginine residue or a lysine residue. The polar uncharged amino acid residue of X⁶ is preferably a threonine residue or a glutamine residue. The acidic amino acid residue of X⁶ is preferably a glutamic acid residue. X⁶ is preferably an arginine residue, a lysine residue, a threonine residue, a glutamine residue, a glutamic acid residue or an alanine residue.

X⁷ is preferably a hydrophobic amino acid residue, a polar uncharged amino acid residue or a basic amino acid residue. The hydrophobic amino acid residue of X⁷ is preferably a phenylalanine residue, a valine residue, a tryptophan residue or a tyrosine residue. The polar uncharged amino acid residue of X⁷ is preferably a cysteine residue. The basic amino acid residue of X⁷ is preferably a histidine residue. X⁷ is preferably a phenylalanine residue, a valine residue, a tryptophan residue, a tyrosine residue, a cysteine residue or a histidine residue.

The amino acid sequence represented by the formula (1) is preferably an amino acid sequence represented by the following formula (2) (SEQ ID NO: 2), more preferably an amino acid sequence represented by the following formula (3) (SEQ ID NO: 3).

LXJPWXXGXXXJ (2)

LXJPWXXGXX^{A}XJ (3)

[In the formula, L represents a leucine residue, P represents a proline residue, W represents a tryptophan residue, G represents a glycine residue, J represents a hydrophobic amino acid residue, X^{A} represents a basic amino acid residue, and X represents any amino acid residue.]

Specific examples of the amino acid sequence represented by the formula (1) include the amino acid sequences of SEQ ID NO: 7, 8, 35, 38, 41, 44, 47, 50, 53, 63, 66, 69, 72, 75 or 78. The amino acid sequence of SEQ ID NO: 7 is an amino acid sequence contained in a cryptochrome (CYME_CMM076C) of C. *merolae.* The amino acid sequence of SEQ ID NO: 8 is an amino acid sequence contained in a cryptochrome (CYME_CMQ453C) of C. *merolae.* The amino acid sequence of SEQ ID NO: 35 is an amino acid sequence contained in QNL10737.1 of *Haematococcus lacustris.* The amino acid sequence of SEQ ID NO: 38 is an amino acid sequence contained in EWM23893 of *Nannochloropsis gaditana.* The amino acid sequence of SEQ ID NO: 41 is an amino acid sequence contained in XP_005852637 of *Nannochloropsis gaditana.* The amino acid sequence of SEQ ID NO: 44 is an amino acid sequence contained in XP_002179379 of *Phaeodactylum tricornutum.* The amino acid sequence of SEQ ID NO: 47 is an amino acid sequence contained in XP_002180095 of *Phaeodactylum tricornutum.* The amino acid sequence of SEQ ID NO: 50 is an amino acid sequence contained in KAG8458188 of *Diacronema lutheri.* The amino acid sequence of SEQ ID NO: 53 is an amino acid sequence contained in KAG8463402 of *Diacronema lutheri.* The amino acid sequence of SEQ ID NO: 63 is an amino acid sequence contained in GENE1 of *Chaetoceros muelleri.* The amino acid sequence of SEQ ID NO: 66 is an amino acid sequence contained in GENE2 of *Chaetoceros muelleri.* The amino acid sequence of SEQ ID NO: 69 is an amino acid sequence contained in GENE3 of *Chaetoceros muelleri.* The amino acid sequence of SEQ ID NO: 72 is an amino acid sequence contained in GENE5 of *Chaetoceros gracilis.* The amino acid sequence of SEQ ID NO: 75 is an amino acid sequence contained in GENE6 of *Chaetoceros gracilis.* The amino acid sequence of SEQ ID NO: 78 is an amino acid sequence contained in GENE7 of *Chaetoceros gracilis.*
LLLPWQIGARCL (SEQ ID NO: 7)
LLIPWQHGLRFL (SEQ ID NO: 8)
LLLPWQWGLKHY (SEQ ID NO: 35)
LWQSWEEGARVF (SEQ ID NO: 38)
LWQSWEEGARVF (SEQ ID NO: 41)
LRVDWTKGAEWF (SEQ ID NO: 44)
LWQSWEDGATVF (SEQ ID NO: 47)
LLVDWRHGARWF (SEQ ID NO: 50)
LYVRWEEGAAVF (SEQ ID NO: 53)
LWQSWEKGAEHF (SEQ ID NO: 63)
LGLSWKDGMQHF (SEQ ID NO: 66)
LHLDWRAGAEWF (SEQ ID NO: 69)
LWQSWEKGAEHF (SEQ ID NO: 72)
LLIDWRWGEAYF (SEQ ID NO: 75)
LGLSWKDGMQHF (SEQ ID NO: 78)

The dark active cryptochrome preferably has a mutation in the amino acid sequence represented by the formula (1) of the dark inactive cryptochrome. Examples of the mutated amino acid residue include the leucine residue (L) at position 1, the tryptophan residue (W) at position 5, the glycine residue (G) at position 8, the leucine residue (L) at position 12 and the like of the amino acid sequence represented by the formula (1). The mutated amino acid residue is preferably the glycine residue (G) at position 8 of the amino acid sequence represented by the formula (1). The mutation is preferably substitution with another amino acid residue.

In the dark active cryptochrome, the glycine residue (G) at position 8 of the amino acid sequence represented by the formula (1) of the dark inactive cryptochrome has been preferably substituted with another amino acid residue. The glycine residue (G) at position 8 of the amino acid sequence represented by the formula (1) has been substituted preferably with a basic amino acid residue. The basic amino acid residue may be an arginine residue (R), a lysine residue (K) or a histidine residue (H). The glycine residue (G) at position 8 of the amino acid sequence represented by the formula (1) has been substituted preferably with an arginine residue (R) or a lysine residue (K) or has been substituted more preferably with an arginine residue (R).

The dark active cryptochrome preferably contains an amino acid sequence represented by the following formula (4), more preferably contains an amino acid sequence represented by the following formula (5) and further preferably contains an amino acid sequence represented by the following formula (6). In the following formulae (4) to (6), X^{A} at position 8 is preferably an arginine residue or a lysine residue, more preferably an arginine residue. In the following formulae (4) to (6), preferable examples of Z, J and X's are the same as those in the formula (1).

LXZXWXXX^{A}XXXJ (4)

LXJPWXXX^{A}XXXJ (5)

LXJPWXXX^{A}XX^{A}XJ (6)

[In the formulae, L represents a leucine residue, P represents a proline residue, W represents a tryptophan residue, G represents a glycine residue, Z represents a hydrophobic amino acid residue or a polar uncharged amino acid residue, J represents a hydrophobic amino acid residue, and X^{A} represents a basic amino acid residue, X represents any amino acid residue.]

The dark active cryptochrome preferably contains the amino acid sequence of SEQ ID NO: 9, 10, 54, 55, 56, 57, 58, 59, 60, 79, 80, 81, 82, 83 or 84.
LLLPWQIRARCL (SEQ ID NO: 9)
LLIPWQHRLRFL (SEQ ID NO: 10)
LLLPWQWRLKHY (SEQ ID NO: 54)
LWQSWEERARVF (SEQ ID NO: 55)
LWQSWEERARVF (SEQ ID NO: 56)
LRVDWTKRAEWF (SEQ ID NO: 57)
LWQSWEDRATVF (SEQ ID NO: 58)
LLVDWRHRARWF (SEQ ID NO: 59)
LYVRWEERAAVF (SEQ ID NO: 60)
LWQSWEKRAEHF (SEQ ID NO: 79)
LGLSWKDRMQHF (SEQ ID NO: 80)
LHLDWRARAEWF (SEQ ID NO: 81)
LWQSWEKRAEHF (SEQ ID NO: 82)
LLIDWRWREAYF (SEQ ID NO: 83)
LGLSWKDRMQHF (SEQ ID NO: 84)

A specific example of the dark active cryptochrome is a polypeptide having an amino acid sequence selected from the group consisting of (a) to (c) below:
(a) an amino acid sequence in which the glycine residue at position 8 of an amino acid sequence represented by the following formula (1) has been substituted with a basic amino acid residue in the amino acid sequence of SEQ ID NO: 12, 14, 34, 37, 40, 43, 46, 49, 52, 62, 65, 68, 71, 74 or 77,

   LXZXWXXGXXXJ (1)

   [in the formula, L represents a leucine residue, W represents a tryptophan residue, G represents a glycine residue, Z represents a hydrophobic amino acid residue or a polar uncharged amino acid residue, J represents a hydrophobic amino acid residue, and X represents any amino acid residue];
(b) an amino acid sequence having a mutation of one amino acid or a plurality of amino acids in the amino acid sequence of SEQ ID NO: 12, 14, 34, 37, 40, 43, 46, 49, 52, 62, 65, 68, 71, 74 or 77 in which the mutation includes a mutation of substituting the glycine residue at position 8 of the amino acid sequence represented by the formula (1) with a basic amino acid residue; and
(c) an amino acid sequence which has a sequence identity of 70% or more with the amino acid sequence of SEQ ID NO: 12, 14, 34, 37, 40, 43, 46, 49, 52, 62, 65, 68, 71, 74 or 77 and in which the glycine residue at position 8 of the amino acid sequence represented by the formula (1) has been substituted with a basic amino acid residue.

In (b) above, the "plurality of amino acids" may be two to 20 residues, two to 15 residues, two to 10 residues, two to nine residues, two to eight residues, two to seven residues, two to six residues, two to five residues, two to four residues, two to three residues, or two residues.

In (c) above, the sequence identity may be 75% or more, 80% or more, 85% or more, 90% or more, 91% or more, 92% or more, 93% or more, 94% or more, 95% or more, 96% or more, 97% or more, 98% or more or 99% or more.

The amino acid sequence of (b) above is preferably an amino acid sequence which has a mutation of one amino acid or a plurality of amino acids in the amino acid sequence of SEQ ID NO: 12, 14, 34, 37, 40, 43, 46, 49, 52, 62, 65, 68, 71, 74 or 77 and which contains the amino acid sequence represented by the formula (4). The amino acid sequence of (c) above is preferably an amino acid sequence which has a sequence identity of 70% or more with the amino acid sequence of SEQ ID NO: 12, 14, 34, 37, 40, 43, 46, 49, 52, 62, 65, 68, 71, 74 or 77 and which contains the amino acid sequence represented by the formula (4). The amino acid sequence represented by the formula (4) is preferably the amino acid sequence represented by the formula (5), preferably the amino acid sequence represented by the formula (6).

In the dark active cryptochrome, the amino acid residue which corresponds to the glycine residue at position 380 (G380) of cryptochrome CRY1 *of Arabidopsis thaliana* (referred to as *"Arabidopsis thaliana* CRY1" below) (SEQ ID NO: 32) in an alignment with *Arabidopsis thaliana* CRY1 may have been substituted with a basic amino acid residue compared to a dark inactive cryptochrome. The basic amino acid is preferably an arginine residue or a lysine residue, more preferably an arginine residue.

A specific example of the dark active cryptochrome is a polypeptide having the amino acid sequence of SEQ ID NO: 16 or 18.

### <<Method for Obtaining Dark Active Cryptochrome Gene>>

The alga having a dark active cryptochrome has a dark active cryptochrome gene which expresses a dark active cryptochrome. The dark active cryptochrome gene may be obtained by introducing a mutation which expresses the dark active cryptochrome into a wild-type cryptochrome gene. As the wild-type cryptochrome gene, a known gene may be used, or a wild-type cryptochrome gene may be newly identified in a desired alga. As the known wild-type cryptochrome gene, for example, those registered in a gene database (GenBank, Uniprot or the like) may be used.

When a wild-type cryptochrome gene is newly identified, the identification method is not particularly limited, and a known method can be used. For example, homology search may be conducted in a protein sequence database (for example, a non-redundant protein sequence database) using the amino acid sequence of an existing wild-type cryptochrome as a query. The homology search can be conducted, for example, using BLASTP. A protein sequence which exhibits, for example, a homology of 20% or more, 21% or more, 22% or more, 23% or more, 24% or more, 25% or more, 26% or more, 27% or more, 28% or more, 29% or more or 30% or more with the amino acid sequence of the wild-type cryptochrome used as the query as a result of homology search may be obtained as a cryptochrome candidate sequence. Examples of the existing wild-type cryptochrome include *Arabidopsis thaliana* CRY1 (SEQ ID NO: 32), CYME_CMM076C (SEQ ID NO: 12) of *C*. *merolae,* CYME_CMQ453C (SEQ ID NO: 14) of *C*. *merolae,* QNL10737.1 (SEQ ID NO: 34) of *H. lacustris* and the like. A gene encoding a protein exhibiting a homology with the wild-type cryptochrome can be obtained as a wild-type cryptochrome candidate gene.

When the genome of the desired alga has not been decoded sufficiently, the genome sequence may be obtained by decoding the genome by a known method. The genome can be decoded, for example, using a next-generation sequencer. Through homology search in the obtained genome sequence using the gene sequence of an existing wild-type cryptochrome as a query, a wild-type cryptochrome candidate gene can be obtained. The homology search can be conducted, for example, using BLASTN. Alternatively, the ORF is estimated from the genome sequence, and the amino acid sequence of the protein encoded by the ORF may be obtained. Through homology search of the amino acid sequence using the amino acid sequence of an existing wild-type cryptochrome as a query in the same manner as described above, a wild-type cryptochrome candidate sequence can be obtained. The ORF encoding the amino acid sequence can be obtained as a wild-type cryptochrome candidate gene.

Alternatively, a cDNA library of the desired alga may be produced, and a cryptochrome candidate gene may be searched. For example, using the full-length cDNA of an existing wild-type cryptochrome or a partial fragment thereof as a probe, search in a cDNA library of the desired alga may be conducted. cDNA which hybridizes with the probe can be obtained as the cDNA of a cryptochrome candidate gene. Examples of the cryptochrome cDNA used as a probe include cDNA of *Arabidopsis thaliana* CRY1 (SEQ ID NO: 31), cDNA of CYME_CMM076C (SEQ ID NO: 11), cDNA of CYME_CMQ453C (SEQ ID NO: 13) of C. *merolae,* cDNA of QNL10737.1 (SEQ ID NO: 33) of *H. lacustris* and the like.

Alternatively, degenerate primers may be designed from the amino acid sequence of an existing wild-type cryptochrome, and degenerate PCR may be conducted using the cDNA library of the desired alga as a template. By analyzing the sequence of the nucleic acid fragment amplified by the degenerate PCR, a partial sequence of a cryptochrome candidate gene can be obtained. Next, inverse primers may be designed from the obtained partial sequence, and inverse PCR may be conducted using the cDNA library as a template. As a result, the full-length cDNA sequence of the wild-type cryptochrome candidate gene can be obtained.

When the obtained cDNA sequence of the wild-type cryptochrome candidate gene is not complete, the full-length cDNA sequence may be obtained using 5' RACE, 3' RACE and the like.

After a wild-type cryptochrome candidate gene of the desired alga is obtained, domain search may be conducted for the protein (candidate protein) encoded by the candidate gene. The protein domain search can be conducted using, for example, a known protein domain database (Pfam or the like). When a candidate gene encodes a protein without the FAD-binding domain as a result of the protein domain search, the candidate gene may be excluded from the wild-type cryptochrome candidate genes.

A protein in which the amino acid residues that correspond to the aspartic acid residue at position 359 (D359) and the valine residue at position 363 (V363) of *Arabidopsis thaliana* CRY1 in an alignment with the amino acid sequence of CRY1 of *Arabidopsis thaliana* (SEQ ID NO: 32) are histidine residues is highly likely a photolyase. Accordingly, a candidate gene encoding such a protein may be excluded from the wild-type cryptochrome candidate genes. BLASTP, Clustal Omega or the like can be used for the alignment.

When a plurality of candidate genes are isolated from the desired alga, the candidate gene which encodes a closer protein to an existing wild-type cryptochrome may be selected as the wild-type cryptochrome gene through a phylogenetic analysis of the amino acid sequences of the proteins encoded by the candidate genes. The existing wild-type cryptochrome may be *Arabidopsis thaliana* CRY1, CYME_CMM076C and CYME_CMQ453C of *C*. *merolae,* QNL1073 7.1 of *H. lacustris* or the like.

A candidate gene encoding a protein containing the amino acid sequence represented by the formula (1) may be selected as a cryptochrome gene. The amino acid sequence represented by the formula (1) is preferably the amino acid sequence represented by the formula (2), more preferably the amino acid sequence represented by the formula (3).

A candidate gene encoding a protein in which the amino acid residue that corresponds to the glycine residue at position 380 (G380) *of Arabidopsis thaliana* CRY1 in an alignment with the amino acid sequence of CRY1 of *Arabidopsis thaliana* (SEQ ID NO: 32) is a glycine residue may be selected as a wild-type cryptochrome gene.

By introducing a mutation which expresses a dark active cryptochrome into the obtained wild-type cryptochrome gene, a dark active cryptochrome gene can be obtained. The mutation can be introduced using a known method. Examples of the method for introducing the mutation include a method for conducting inverse PCR using primers with the mutation introduced at the desired position and the like. A commercial mutation kit may be used for introducing the mutation.

The site for introducing the mutation may be the site described above. The mutation-introduced site is preferably the FAD-binding domain, more preferably the amino acid sequence represented by the formula (1), further preferably the glycine residue at position 8 of the amino acid sequence represented by the formula (1). The mutation-introduced site may be the amino acid residue which corresponds to the glycine residue at position 380 (G380) *of Arabidopsis thaliana* CRY1 in an alignment with the amino acid sequence of CRY1 *of Arabidopsis thaliana* (SEQ ID NO: 32).

That the obtained gene expresses a dark active cryptochrome can be checked by introducing the gene into an alga and heterotrophically culturing in the dark. When the gene expresses a dark active cryptochrome, the heterotrophic growth in the dark is improved compared to that of the original alga without the introduction of the gene. Examples of the improvement of the heterotrophic growth include improvement of the growth rate in heterotrophic cultivation, improvement of the maximum reached algal density in heterotrophic cultivation, improvement of the growth rate after subculture in heterotrophic cultivation and the like.

The alga which has been modified to express a dark active cryptochrome may have a specific growth rate that is 1.2 times or more, 1.5 times or more, 1.8 times or more, 2 times or more, 2.2 times or more, 2.5 times or more, 2.7 times or more, 3 times or more, 3.2 times or more, 3.5 times or more, 3.7 times or more, 4 times or more, 4.2 times or more, 4.5 times or more, 4.7 times or more or 5 times or more higher than that of the original alga in heterotrophic cultivation under the same conditions.

The alga which has been modified to express a dark active cryptochrome may have a maximum reached algal density that is 1.2 times or more, 1.5 times or more, 1.8 times or more, 2 times or more, 2.2 times or more, 2.5 times or more, 2.7 times or more, 3 times or more, 3.2 times or more, 3.5 times or more, 3.7 times or more, 4 times or more, 4.2 times or more, 4.5 times or more, 4.7 times or more or 5 times or more higher than that of the original alga in heterotrophic cultivation under the same conditions.

The specific growth rate after subculture of the alga which has been modified to express a dark active cryptochrome may be 1.2 times or more, 1.5 times or more, 1.8 times or more, 2 times or more, 2.2 times or more, 2.5 times or more, 2.7 times or more, 3 times or more, 3.2 times or more, 3.5 times or more, 3.7 times or more, 4 times or more, 4.2 times or more, 4.5 times or more, 4.7 times or more or 5 times or more higher than that of the original alga when the algae are subcultured in heterotrophic cultivation under the same conditions.

As described above, the gene which has been found to express a dark active cryptochrome can be obtained as the dark active cryptochrome gene. By introducing the dark active cryptochrome gene into an alga, an alga having a dark active cryptochrome can be obtained.

The alga of the embodiment can be cultured in a medium which is appropriately selected depending on the species of the alga. The alga of the embodiment can be cultured, for example, using a known medium for algae. An example of the medium is an inorganic salt medium containing, for example, a nitrogen source, a phosphorus source, a trace element (zinc, boron, cobalt, copper, manganese, molybdenum, iron or the like) and the like. The nitrogen source may be an ammonium salt, a nitrate, a nitrite or the like, and the phosphorus source may be a phosphate or the like. Examples of such an inorganic salt medium include Gross medium, 2× Allen medium (Allen MB. Arch. Microbiol. 1959 32: 270-277.), M-Allen medium (Minoda A et al. Plant Cell Physiol. 2004 45: 667-71.), MA2 medium (Ohnuma M et al. Plant Cell Physiol. 2008 Jan; 49(1): 117-20.), modified M-Allen medium and the like, but the medium is not limited to the examples.

In the case of heterotrophic cultivation, an organic carbon source may be added to the inorganic salt medium described above. Examples of the organic carbon source include a sugar alcohol, a saccharide, an amino acid and the like. An example of the sugar alcohol is glycerol. Examples of the saccharide include glucose, mannose, fructose, sucrose, maltose and lactose saccharide. The concentration of the organic carbon source in the medium is, for example, 0.1 to 10% (w/v) or 0.1 to 10% (v/v).

The cultivation conditions of the alga of the embodiment are not particularly limited and can be appropriately selected depending on the species of the alga. The cultivation conditions are, for example, pH 1 to 8, a temperature of 10 to 50°C and a CO₂ concentration of 0.3 to 3% and the like.

The cultivation conditions are not limited to the conditions exemplified above and can be appropriately selected depending on the species of the alga. For example, when the alga is of Cyanidiophyceae, the pH conditions may be pH 1.0 to 6.0 and are preferably pH 1.0 to 5.0, more preferably pH 1.0 to 3.0. The temperature conditions may be 15 to 50°C and are preferably 30 to 50°C, more preferably 35 to 50°C. In the case of autotrophic cultivation or mixotrophic cultivation, the light intensity may be 5 to 2000 µmol/m²s and is preferably 5 to 1500 µmol/m²s. In the case of autotrophic cultivation or mixotrophic cultivation, the alga may be cultured in continuous light, or the alga may be cultured in a light-dark cycle (10L: 14D or the like). In heterotrophic cultivation, the alga may be cultured in the dark.

Because the alga of the embodiment has a dark active cryptochrome, the heterotrophic growth in the dark is improved compared to that of the alga having a dark inactive cryptochrome only. Accordingly, the alga can be cultured efficiently with a high density also in the dark. Due to such properties, the alga is believed to be suitable for an industrial application.

### <Improvement Method of Growth of Alga in the Dark>

A second aspect of the invention is a method for improving growth of an alga in the dark including introducing a polynucleotide encoding a dark active cryptochrome into an alga.

### (Alga)

The alga is not particularly limited, and any alga can be used. The alga may be the same alga as the algae listed in the section <Alga> above.

### (Polynucleotide Encoding Dark Active Cryptochrome)

The polynucleotide encoding a dark active cryptochrome is not particularly limited as long as the polynucleotide contains a nucleotide sequence encoding a dark active cryptochrome (a dark active cryptochrome-encoding sequence). The polynucleotide encoding a dark active cryptochrome is a polynucleotide containing a dark active cryptochrome gene. The polynucleotide encoding a dark active cryptochrome is a polynucleotide containing a dark active cryptochrome gene. The dark active cryptochrome gene can be obtained by the method described in <Alga> above. A specific example of the dark active cryptochrome gene is a polynucleotide containing the nucleotide sequence of SEQ ID NO: 15 or 17.

The polynucleotide encoding a dark active cryptochrome may have another sequence in addition to the dark active cryptochrome gene (the dark active cryptochrome-encoding sequence). Examples of the other sequence include a sequence which regulates the expression of the dark active cryptochrome gene and the like. Examples of the sequence regulating the expression of the dark active cryptochrome gene include a promoter, an enhancer, a poly(A) addition signal, a terminator and the like, but the sequence is not limited to the examples.

The dark active cryptochrome gene may be functionally linked to any promoter. "Functionally linked" means that a first base sequence is located close enough to a second base sequence and that the first base sequence can influence the second base sequence. For example, that a gene is functionally linked to a promoter means that the gene is linked in such a manner that the gene is expressed under the regulation of the promoter. A promoter which can function in the algal cells can be appropriately selected depending on the species of the alga. The promoter may be the promoter of the wild-type cryptochrome gene or may be the promoter of another gene. Examples of the promoter of another gene include APCC promoter, CPCC promoter, Catalase promoter, EF1α promoter and the like, but the promoter is not limited to the examples. The promoter may be the promoter of another organism (for example, an alga of another species).

Any terminator may be linked to the 3' end of the dark active cryptochrome gene. A terminator which can function in the algal cells can be appropriately selected depending on the species of the alga. The terminator may be the terminator of the wild-type cryptochrome gene or the terminator of another gene. The terminator of another gene may be APCC terminator, CPCC terminator, Catalase terminator, EF1α terminator, β-tubulin terminator, ubiquitin terminator or the like but is not limited thereto. The terminator may be the terminator of another organism (for example, an alga of another species).

The polynucleotide encoding a dark active cryptochrome may contain a selective marker gene in addition to the dark active cryptochrome gene. Examples of the selective marker gene include an antibiotic resistance gene, a gene relevant to auxotrophy, a fluorescent protein gene and the like. Examples of the antibiotic resistance gene include a blasticidin S resistance gene, a chloramphenicol resistance gene, an ampicillin resistance gene, a kanamycin resistance gene, a tetracycline resistance gene and the like. Examples of the gene relevant to auxotrophy include URA5.3 gene and the like. Examples of the fluorescent protein gene include mVenus gene, GFP gene, mCherry gene and the like. These selective marker genes may have an expression-regulating sequence such as a promoter, an enhancer, a poly(A) addition signal, a terminator and the like. The marker gene may be functionally linked to a promoter which can function in the algal cells.

When the dark active cryptochrome gene is derived from an organism species other than the alga of the subject of introduction (host alga), the codons of the nucleotide sequence of the dark active cryptochrome gene may be optimized based on the codon usage frequency of the host alga. Through codon optimization, the translation efficiency into the dark active cryptochrome in the host alga can be improved.

The polynucleotide encoding a dark active cryptochrome may contain a 5' homology arm and a 3' homology arm. The 5' homology arm and the 3' homology arm contain a nucleotide sequence of the target region to which the dark active cryptochrome gene is introduced in the host alga. The 5' homology arm can contain a nucleotide sequence adjacent to the 5' side of the target region. The 3' homology arm can contain a nucleotide sequence adjacent to the 3' side of the target region. The sizes of the 5' homology arm and the 3' homology arm may be sizes which cause homologous recombination with the genome DNA of the host alga and are not particularly limited. The 5' homology arm and the 3' homology arm can be, for example, of approximately 500 to 3000 bp.

When the polynucleotide encoding a dark active cryptochrome contains the 5' homology arm and the 3' homology arm, the 5' homology arm can be placed at the 5' side (upstream side) of the dark active cryptochrome gene (or the dark active cryptochrome gene and the marker gene). The 3' homology arm can be placed at the 3' side (downstream side) of the dark active cryptochrome gene (or the dark active cryptochrome gene and the marker gene).

### (Introduction Method of Polynucleotide Encoding Dark Active Cryptochrome)

The method for introducing the polynucleotide encoding a dark active cryptochrome into algal cells is not particularly limited, and a known method can be used. Examples of the method for introducing the polynucleotide encoding a dark active cryptochrome into algal cells include a method using genome editing, homologous recombination and the like.

The method using genome editing may be a method using a genome editing system containing a sequence-specific endonuclease. The "genome editing system containing a sequence-specific endonuclease" means a system in which the genome DNA can be cleaved sequence specifically with a sequence-specific endonuclease and a mutation can be induced in the cleaved region.

The sequence-specific endonuclease is an enzyme which can cleave a nucleic acid at a certain sequence. The sequence-specific endonuclease is preferably a sequence-specific endodeoxyribonuclease which can cleave double-strand DNA at a certain sequence. Examples of the sequence-specific endonuclease include zinc finger nuclease (ZFN), TALEN (transcription activator-like effector nuclease), Cas protein and the like, but the sequence-specific endonuclease is not limited to the examples. The genome editing system is preferably Cas protein-using CRISPR (Clustered Regularly Interspaced Short Palindromic Repeat)/Cas system.

When genome editing is used, the polynucleotide encoding a dark active cryptochrome can be introduced into the algal cells as doner DNA after the genome DNA cleavage of the target region by the sequence-specific endonuclease or together with the sequence-specific endonuclease. The doner DNA preferably contains a 5' homology arm and a 3' homology arm. By introducing the doner DNA into the algal cells after the cleavage of the genome DNA by the sequence-specific endonuclease or together with the sequence-specific endonuclease, homologous directed repair (HDR) is induced, and the dark active cryptochrome gene (or the dark active cryptochrome gene and the marker gene) is incorporated into the target region.

The homologous recombination method is a genome modification method using the phenomenon of recombination caused between two DNA double strands having homologous sequences. In the homologous recombination method, a targeting vector can be used. The targeting vector can contain the dark active cryptochrome gene (or the dark active cryptochrome gene and the marker gene), a 5' homology arm and a 3' homology arm. By introducing the targeting vector into the algal cells, the dark active cryptochrome gene (or the dark active cryptochrome gene and the marker gene) is incorporated into the genome DNA of the algal cells through homologous recombination.

When being introduced into the algal cells, the polynucleotide encoding a dark active cryptochrome may be a linear DNA vector or a cyclic DNA vector but is preferably a linear DNA vector. The method for introducing the polynucleotide encoding a dark active cryptochrome into the algal cells is not particularly limited, and a known method can be used. Examples of the introduction method include polyethylene glycol method, lipofection method, microinjection method, DEAE dextran method, particle gun method, electroporation method, calcium phosphate method and the like.

The algal cells after the operation of polynucleotide introduction may be cultured, and cells into which the dark active cryptochrome gene has been introduced may be selected. When a marker gene is introduced with the dark active cryptochrome gene, the gene-introduced cells can be selected based on the expression of the marker gene. When the marker gene is an antibiotic resistance gene, for example, the cells into which the dark active cryptochrome gene has been introduced can be selected by culturing the algal cells in a medium containing the antibiotic to which the antibiotic resistance gene is resistant. When the marker gene is a gene which recovers the auxotrophy, for example, the cells into which the dark active cryptochrome gene has been introduced can be selected by culturing the algal cells in a medium which does not contain the recovered auxotrophic component. When the marker gene is a fluorescent protein gene, for example, the cells into which the dark active cryptochrome gene has been introduced can be selected by selecting the algal cells using flow cytometry or the like based on the fluorescence.

The cells into which the dark active cryptochrome gene has been introduced may be selected with colony PCR. The colony PCR can be conducted using primers designed based on the nucleotide sequence of the dark active cryptochrome gene. Through the colony PCR, a colony from which the amplification of the DNA fragment is observed is selected, and the cells forming the colony can be selected as the dark active cryptochrome gene-introduced cells.

The alga into which the dark active cryptochrome gene has been introduced has the dark active cryptochrome due to the expression of the dark active cryptochrome gene. Because the alga has the dark active cryptochrome, heterotrophic growth in the dark is improved. Examples of the improvement of the heterotrophic growth in the dark include improvement of the heterotrophic growth rate in the dark, improvement of the maximum reached algal density in heterotrophic cultivation in the dark, improvement of the growth rate after subculture in heterotrophic cultivation in the dark and the like.

By the method of the embodiment, the heterotrophic growth of the alga in the dark can be improved. The improvement of the heterotrophic growth includes that an alga which cannot grow heterotrophically in the dark becomes capable of growing in the dark. By the method of the embodiment, the heterotrophic growth in the dark of a desired alga can be improved.

### <Production Method of Alga with Improved Growth in the Dark>

A third aspect of the invention is a method for producing an alga with improved growth in the dark including introducing a polynucleotide encoding a dark active cryptochrome into an alga.

The alga is not particularly limited, and any alga can be used. The same alga as those listed in the section <Alga> above can be used.

The same polynucleotide encoding a dark active cryptochrome as that described in the section <Improvement Method of Growth of Alga in the Dark> above can be used. The method for introducing the polynucleotide encoding a dark active cryptochrome into an alga can be conducted in the same manner as the method described in the section <Improvement Method of Growth of Alga in the Dark> above.

By the production method of the embodiment, an alga with improved heterotrophic growth in the dark can be obtained. The improvement of the heterotrophic growth in the dark may be the same as that described above.

### <Dark Active Cryptochrome>

A fourth aspect of the invention is a dark active cryptochrome whose activation is maintained in the dark, having an amino acid sequence selected from the group consisting of (a) to (c) below:
(a) an amino acid sequence in which the glycine residue at position 8 of an amino acid sequence represented by the following formula (1) has been substituted with a basic amino acid residue in the amino acid sequence of SEQ ID NO: 12, 14, 34, 37, 40, 43, 46, 49, 52, 62, 65, 68, 71, 74 or 77,

   LXZXWXXGXXXJ (1)

   [in the formula, L represents a leucine residue, W represents a tryptophan residue, G represents a glycine residue, Z represents a hydrophobic amino acid residue or a polar uncharged amino acid residue, J represents a hydrophobic amino acid residue, and X represents any amino acid residue];
(b) an amino acid sequence having a mutation of one amino acid residue or a plurality of amino acid residues in the amino acid sequence of SEQ ID NO: 12, 14, 34, 37, 40, 43, 46, 49, 52, 62, 65, 68, 71, 74 or 77 in which the mutation includes a mutation of substituting the glycine residue at position 8 of the amino acid sequence represented by the formula (1) with a basic amino acid residue; and
(c) an amino acid sequence which has a sequence identity of 70% or more with the amino acid sequence of SEQ ID NO: 12, 14, 34, 37, 40, 43, 46, 49, 52, 62, 65, 68, 71, 74 or 77 and in which the glycine residue at position 8 of the amino acid sequence represented by the formula (1) has been substituted with a basic amino acid residue.

The basic amino acid residue is preferably an arginine residue or a lysine residue, more preferably an arginine residue.

A specific example of the amino acid sequence of (a) above is the amino acid sequence of SEQ ID NO: 16 or 18.

In (b) above, the "plurality" may be two to 20 residues, two to 15 residues, two to 10 residues, two to nine residues, two to eight residues, two to seven residues, two to six residues, two to five residues, two to four residues, two or three residues or two residues. The "mutation" may be any of deletion, substitution, addition and insertion or may be a combination thereof.

In (c) above, the sequence identity may be 75% or more, 80% or more, 85% or more, 90% or more, 91% or more, 92% or more, 93% or more, 94% or more, 95% or more, 96% or more, 97% or more, 98% or more or 99% or more.

Preferable specific examples of (b) and (c) above are the same as those described in the section <Alga> above.

### <Polynucleotide Encoding Dark Active Cryptochrome>

A fifth aspect of the invention is a polynucleotide encoding the dark active cryptochrome of the fourth aspect.

A specific example of the polynucleotide of the embodiment is a polynucleotide containing a nucleotide sequence selected from the group consisting of (d) to (f) below.

(d) A nucleotide sequence in which the codon encoding the glycine residue at position 8 of the amino acid sequence represented by the formula (1) has been substituted with a codon encoding a basic amino acid residue in the nucleotide sequence of SEQ ID NO: 11, 13, 33, 36, 39, 42, 45, 48, 51, 61, 64, 67, 70, 73 or 76.

(e) A nucleotide sequence which has a mutation of one nucleotide residue or a plurality of nucleotide residues in the nucleotide sequence of SEQ ID NO: 11, 13, 33, 36, 39, 42, 45, 48, 51, 61, 64, 67, 70, 73 or 76 and which contains a nucleotide sequence encoding an amino acid sequence in which the glycine residue at position 8 of the amino acid sequence represented by the formula (1) has been substituted with a basic amino acid residue.

(f) A nucleotide sequence which has a sequence identity of 70% or more with the nucleotide sequence of SEQ ID NO: 11, 13, 33, 36, 39, 42, 45, 48, 51, 61, 64, 67, 70, 73 or 76 and which contains a nucleotide sequence encoding an amino acid sequence in which the glycine residue at position 8 of the amino acid sequence represented by the formula (1) has been substituted with a basic amino acid residue.

The polynucleotide of the embodiment may be a polynucleotide of (g) below.
(g) A nucleotide sequence which is a polynucleotide that hybridizes with a polynucleotide having the nucleotide sequence of SEQ ID NO: 11, 13, 33, 36, 39, 42, 45, 48, 51, 61, 64, 67, 70, 73 or 76 under stringent conditions and which contains a nucleotide sequence encoding an amino acid sequence in which the glycine residue at position 8 of the amino acid sequence represented by the formula (1) has been substituted with a basic amino acid residue.

The basic amino acid residue is preferably an arginine residue or a lysine residue, more preferably an arginine residue.

Specific examples of the nucleotide sequence of (d) above include: a nucleotide sequence in which the nucleotide sequence at positions 1255 to 1257 has been substituted with a codon encoding a basic amino acid residue in the nucleotide sequence of SEQ ID NO: 11; a nucleotide sequence in which the nucleotide sequence at positions 1714 to 1716 has been substituted with a codon encoding a basic amino acid residue in the nucleotide sequence of SEQ ID NO: 13; a nucleotide sequence in which the nucleotide sequence at positions 1156 to 1158 has been substituted with a codon encoding a basic amino acid residue in the nucleotide sequence of SEQ ID NO: 33; a nucleotide sequence in which the nucleotide sequence at positions 1336 to 1338 has been substituted with a codon encoding a basic amino acid residue in the nucleotide sequence of SEQ ID NO: 36; a nucleotide sequence in which the nucleotide sequence at positions 1243 to 1245 has been substituted with a codon encoding a basic amino acid residue in the nucleotide sequence of SEQ ID NO: 39; a nucleotide sequence in which the nucleotide sequence at positions 1201 to 1203 has been substituted with a codon encoding a basic amino acid residue in the nucleotide sequence of SEQ ID NO: 42; a nucleotide sequence in which the nucleotide sequence at positions 1222 to 1224 has been substituted with a codon encoding a basic amino acid residue in the nucleotide sequence of SEQ ID NO: 45; a nucleotide sequence in which the nucleotide sequence at positions 1318 to 1320 has been substituted with a codon encoding a basic amino acid residue in the nucleotide sequence of SEQ ID NO: 48; a nucleotide sequence in which the nucleotide sequence at positions 1207 to 1209 has been substituted with a codon encoding a basic amino acid residue in the nucleotide sequence of SEQ ID NO: 51; a nucleotide sequence in which the nucleotide sequence at positions 259 to 261 has been substituted with a codon encoding a basic amino acid residue in the nucleotide sequence of SEQ ID NO: 61; a nucleotide sequence in which the nucleotide sequence at positions 1579 to 1581 has been substituted with a codon encoding a basic amino acid residue in the nucleotide sequence of SEQ ID NO: 64; a nucleotide sequence in which the nucleotide sequence at positions 1567 to 1569 has been substituted with a codon encoding a basic amino acid residue in the nucleotide sequence of SEQ ID NO: 67; a nucleotide sequence in which the nucleotide sequence at positions 259 to 261 has been substituted with a codon encoding a basic amino acid residue in the nucleotide sequence of SEQ ID NO: 70; a nucleotide sequence in which the nucleotide sequence at positions 1939 to 1041 has been substituted with a codon encoding a basic amino acid residue in the nucleotide sequence of SEQ ID NO: 73; and a nucleotide sequence in which the nucleotide sequence at positions 1600 to 1602 has been substituted with a codon encoding a basic amino acid residue in the nucleotide sequence of SEQ ID NO: 76. An example is the nucleotide sequence of SEQ ID NO: 15 or 17.

In (e) above, the "plurality" may be two to 20 residues, two to 15 residues, two to 10 residues, two to nine residues, two to eight residues, two to seven residues, two to six residues, two to five residues, two to four residues, two or three residues or two residues. The "mutation" may be any of deletion, substitution, addition and insertion or may be a combination thereof.

In (f) above, the sequence identity may be 75% or more, 80% or more, 85% or more, 90% or more, 91% or more, 92% or more, 93% or more, 94% or more, 95% or more, 96% or more, 97% or more, 98% or more or 99% or more.

In (g) above, the "stringent conditions" mean conditions under which two polynucleotides having a high sequence identity can specifically hybridize. The two polynucleotides having a high sequence identity mean that the sequence identity of the two polynucleotides is, for example, 80% or more, 85% or more, 90% or more, 95% or more, 96% or more, 97% or more, 98% or more or 99% or more. Specific examples of the stringent conditions are, for example, the conditions described in Molecular Cloning-A LABORATORY MANUAL THIRD EDITION (Sambrook et al., Cold Spring Harbor Laboratory Press) and the like. The stringent conditions are, for example, conditions of incubating at 42 to 70°C for several hours to one night in a hybridization buffer containing 6× SSC (the composition of 20× SSC: 3M sodium chloride and 0.3 M citric acid solution, pH 7.0), 5× Denhardt's solution (the composition of 100× Denhardt's solution: 2 mass% bovine serum albumin, 2 mass% Ficoll and 2 mass% polyvinylpyrrolidone), 0.5 mass% SDS, 0.1 mg/mL salmon sperm DNA and 50% formamide. Examples of the wash buffer used for washing after the incubation include 0.1 mass% SDS-containing 1× SSC solution and 0.1 mass% SDS-containing 0.1× SSC solution.

In (e) to (g) above, the "amino acid sequence in which the glycine residue at position 8 of the amino acid sequence represented by the formula (1) has been substituted with a basic amino acid residue" is preferably the amino acid sequence represented by the formula (4), more preferably the amino acid sequence represented by the formula (5), further preferably the amino acid sequence represented by the formula (6).

The polynucleotide of the embodiment can be obtained by introducing a mutation into the gene of a wild-type cryptochrome of an alga. For example, the gene of a wild-type cryptochrome of *C*. *merolae* (for example, CYME_CMM076C or CYME_CMQ453C) can be obtained by a nucleic acid amplification method such as PCR using the primers described in the Examples below and the like using the genome DNA of *C*. *merolae* as the template or another method.

By introducing the polynucleotide of the embodiment into an alga, an alga with improved heterotrophic growth in the dark can be obtained. The alga into which the polynucleotide of the embodiment is introduced is preferably an alga belonging to Cyanidiophyceae, more preferably *C*. *merolae.*

### EXAMPLES

The invention is explained below with Examples, but the invention is not limited to the following Examples.

### <Search of Cryptochrome (CRY) Candidate Genes in C. merolae>

CRY candidate genes were obtained through protein-protein BLAST search of a genome database of *C*. *merolae* strain 10D (Database: non-redundant protein sequences, Organism: the genome of *C*. *merolae* strain 10D (taxid: 280699)) using the amino acid sequence of CRY1 gene *of Arabidopsis thaliana* as a query. No homology with CRY1 was found in the C-terminal region in all the obtained CRY candidate genes. Percent identity (sequence identities) between the obtained CRY candidate genes and CRY1 were 28.6% to 44.44%.

The protein domains were searched in the amino acid sequences encoded by the CRY candidate genes using the Pfam database (EMBL-EBI, UK). Based on the results, the candidate genes without the FAD-binding domain, which is believed to be essential for the CRY function, were excluded. Based on the results of an alignment with *Arabidopsis thaliana* CRY1 using BLAST search, the candidate genes in which the amino acid residues corresponding to D359 and V363 of CRY1 were histidine residues were believed to be (6-4)DNA photolyases and thus were excluded. It has been found that DNA photolyases have a His-His-Leu-Ala-Arg-His motif as a necessary site for the activity (K. Hitomi et al., Proc.Natl.Acad.Sci.U.S.A. (2009)) and that the enzymatic activity is lost when the second histidine residue and the last histidine residue in the motif are mutated. The histidine residues correspond to the amino acid residues at position 359 and at position 363 of *Arabidopsis thaliana* CRY1. In the amino acid sequences encoded by the remaining CRY candidate genes, the amino acid residue corresponding to G380 of CRY1 was conserved and remained to be the glycine residue. In the amino acid sequences encoded by the CRY candidate genes, the amino acid sequence of the following formula (1) was conserved as a motif containing the glycine residue.

LXZXWXXGXXXJ (1)

[In the formula, L represents a leucine residue, W represents a tryptophan residue, G represents a glycine residue, Z represents a hydrophobic amino acid residue or a polar uncharged amino acid residue, J represents a hydrophobic amino acid residue, and X represents any amino acid residue.]

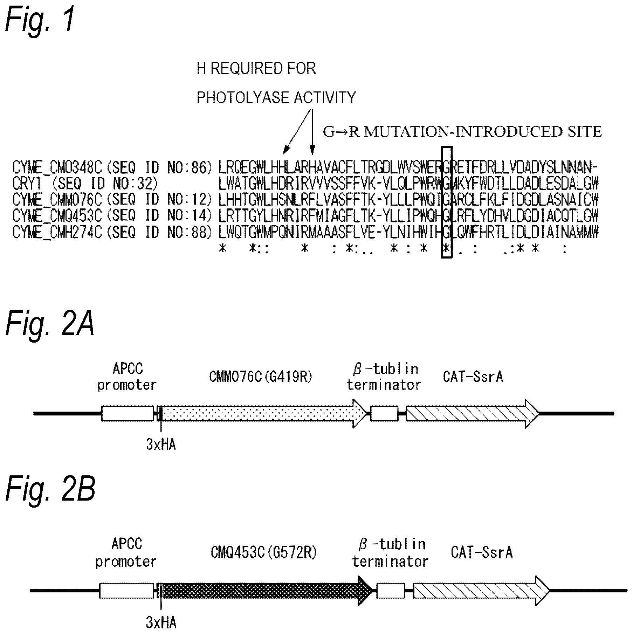

To select closer candidate genes to *Arabidopsis thaliana* CRY1 gene from the CRY candidate genes, a phylogenetic analysis was conducted using Clustal Omega (EMBL-EBI, UK). As a result, two final candidate genes (CYME_CMM076C and CYME_CMQ453C) were obtained. The nucleotide sequence and the amino acid sequence of CYME_CMM076C are shown in SEQ ID NO: 11 and SEQ ID NO: 12, respectively. The nucleotide sequence and the amino acid sequence of CYME_CMQ453C are shown in SEQ ID NO: 13 and SEQ ID NO: 14, respectively. The results of the alignment using Clustal Omega are shown in Fig. 1. In Fig. 1, the amino acid sequences around the motif of the formula (1) are shown. In Fig. 1, "G's" in the square are G380 *of Arabidopsis thaliana* CRY1 and the corresponding glycine residues.

### <Preparation of Linear DNA for Transformation>

A sample for PCR was prepared using KOD One (registered trademark) PCR Master Mix (KMM-201, TOYOBO, Japan) using a suspension containing a trace amount of *C*. *merolae* as a crude sample. In accordance with the protocol provided by the manufacturer, PCR reaction was conducted with a thermal cycler, and the nucleic acid fragments of the candidate genes (CYME_CMM076C gene and CYME_CMQ453C gene) were amplified. The sequences of the primers used are shown below. The capital letters show the sequences corresponding to the genes.
CYME_CMM076C
   Forward primer: gttccagattacgctATGTCGTCGTCGAGACG (SEQ ID NO: 19)
   Reverse primer: taaatagctagtttaTCAGGGCGCGGCAC (SEQ ID NO: 20)
CYME_CMQ453
   Forward primer: gttccagattacgctATGCCCGCGTATCCTC (SEQ ID NO: 21)
   Reverse primer: taaatagctagtttaTCATCTGCGCTTGTCGTC (SEQ ID NO: 22)

After purifying the amplified nucleic acid fragments, the amplified nucleic acid fragments were ligated into a linearized plasmid vector by PCR using In-Fusion (registered trademark) HD Cloning Kit (Z9648N, Takara Bio, Japan) in accordance with the protocol provided by the manufacturer. As the plasmid, one obtained by modifying pGEM-T Easy Vector (A1360, Promega, USA) in such a manner that the inserted gene would be expressed as a protein having 3xHA tag at the N-terminal in the cells of *C*. *merolae* was used (T. Fujiwara et al., Plant Direct. 2019 Apr 8;3(4): e00134.). In this regard, however, CAT-SsrA gene containing chloroplast transit peptide at the N-terminal was used as the selective marker instead of URA gene.

*E. coli* HST08 Premium Competent Cells (#9128, Takara, Japan) were transformed using the In-Fusion reaction solutions containing the linear plasmid vectors into which the amplified nucleic acid fragments were ligated. The cells after the transformation were cultured for a day and a night on LB/Amp plates, and transformed cells were selected. Colonies formed on the plates were subjected to colony direct PCR using KOD One (registered trademark) PCR Master Mix using the primer sets of the candidate genes. In this manner, colonies having the candidate genes were identified. The identified colonies were cultured for a day and a night in a LB/Amp liquid medium. After the cultivation, the bacterial cells were collected from the culture solutions, and the plasmids were purified using a plasmid extraction kit.

Next, using the plasmids purified above, the mutation of substituting the glycine residue corresponding to G380 *of Arabidopsis thaliana* CRY1 (G419 of CYME_CMM076C and G572 of CYME_CMQ453C) with an arginine residue was introduced. The mutation was introduced into each candidate gene using KOD-Plus-Mutagenesis Kit (SMK-101, TOYOBO, Japan). The sequences of the primers used for introducing the mutation are shown below.
CYME_CMM076C
   Forward primer: GGCAGATCCGCGCTCGTTG (SEQ ID NO: 23)
   Reverse primer: ACGGCAGGAGAAGGTACTTGG (SEQ ID NO: 24)
CYME_CMQ453C
   Forward primer: GCCTCCGCTTTCTTTACGATCATG (SEQ ID NO: 25)
   Reverse primer: ATGCTGCCACGGAATGAGAAG (SEQ ID NO: 26)

After the introduction of the mutation, the plasmids were purified using a plasmid extraction kit. PCR reaction was conducted using the purified plasmids as templates using KOD One (registered trademark) PCR Master Mix, and the nucleic acid fragments of the candidate genes into which the mutation was introduced were amplified. The sequences of the primers used are shown below.
Forward primer: CGCCAGGGTTTTCCCAGTCACGAC (SEQ ID NO: 27)
Reverse primer: GAGCGGATAACAATTTCACACAGG (SEQ ID NO: 28)

The amplified nucleic acid fragments were purified using a plasmid extraction kit. The purified nucleic acid fragments were used as linear DNA for transformation. The structure of the linear DNA for transformation obtained by introducing the mutation of G419R into CYME_CMM076C is shown in Fig. 2A. The structure of the linear DNA for transformation obtained by introducing the mutation of G572R into CYME CMQ453C is shown in Fig. 2B. The nucleotide sequence and the amino acid sequence of the mutated gene (CMM076C (G419R)) obtained by introducing the mutation of G419R into CYME_CMM076C are shown in SEQ ID NO: 15 and SEQ ID NO: 16, respectively. The nucleotide sequence and the amino acid sequence of the mutated gene (CMQ453C (G572R)) obtained by introducing the mutation of G572R into CYME CMQ453C are shown in SEQ ID NO: 17 and SEQ ID NO: 18, respectively.

### transformation of C. merolae>

*C. merolae* strain NEIS-1804 (the wild-type strain) was inoculated in 40 mL of MA2 medium (40 mM (NH₄)₂SO₄, 4 mM MgSO₄, 8 mM KH₂PO₄, 1 mM CaCl₂, 0.03 v/v% H₂SO₄, 2.8 µM ZnCl₂, 0.7 µM CoCl₂, 36 µM MnCl₂, 1.3 µM CuCl₂, 6.4 µM Na₂MoO₄ and 4 mL/L-medium 250x Fe Solution [79 mM FeCl₃, 106 mM Na₂EDTA and a few drops of H₂SO₄]) to OD₇₅₀=0.3. Strain NEIS-1804 was cultured photoautotrophically at 40°C with aeration of 600 mL/min air for approximately a day and a night to achieve around OD₇₅₀=0.6. To the culture solution, 1 µL of 20% Tween-20 was added, and after centrifugation for five minutes at room temperature at 2000 xg, the supernatant was removed. The pellet was suspended in MA2 medium, and the suspension was centrifuged again for five minutes at room temperature at 2000 xg. After the supernatant was removed with a micropipette, the pellet was suspended in MA2 medium at OD₇₅₀=100 (wild-type strain suspension).

PEG solution in a volume of 67.5 µL was added to 50 µL of solutions containing 10 to 15 µg of the linear DNA for transformation, and the solutions were mixed. The PEG solution was prepared by adding 80 µL of TF solution [400 mM (NH₄)₂SO₄, 40 mM MgSO₄ and 0.3 v/v% H₂SO₄] to 450 µL of MA2 medium in which 0.4 g of PEG-4000 was dissolved. To the linear DNA solutions for transformation to which the PEG solution was added, 12.5 µL of the wild-type strain suspension was added, and the solutions were mixed by shaking. The suspensions after mixing were transferred to a 6-well plate containing 8 mL/well of MA2 medium with a micropipette and mixed with the medium in the wells. The 6-well plate and AnaeroPack·CO₂ (Mitsubishi Gas Chemical, Japan) were placed in a sealable container, and the container was sealed, followed by photoautotrophic cultivation at 40°C for two days. The culture solutions were centrifuged for five minutes at room temperature at 2000 xg, and the supernatants were removed. The pellets were suspended in 4 mL of MA2 medium and transferred to a 24-well plate, and a chloramphenicol solution was added to 200 µg/mL. The 24-well plate and AnaeroPack·CO₂ were placed in a sealable container, and the container was sealed, followed by photoautotrophic cultivation at 40°C for approximately two weeks. Green cells which grew in the presence of chloramphenicol were recovered and diluted 3000-to 30000-fold with MA2 medium. These were spotted on MA2 gellan gum plates. The MA2 gellan gum plates and AnaeroPouch·CO₂ (Mitsubishi Gas Chemical, Japan) were placed in a sealable container, and the container was sealed, followed by photoautotrophic cultivation at 40°C for approximately two weeks. Parts of the formed colonies were suspended in 40 µL of ddH₂O. Transformed cells into which the target genes were introduced were obtained by PCR reaction using the colony suspensions as templates using KOD One (registered trademark) PCR Master Mix. The sequences of the primers used are shown below.
Forward primer: TCTGCTCGGCTTAGTTTCGAAAG (SEQ ID NO: 29)
Reverse primer: TACCCCGATTCGATCATACGAGT (SEQ ID NO: 30)

### <Evaluation of Transformed Strains>

The heterotrophic growth in the dark of the transformed strains into which CMM076C (G419R) or CMQ453C (G572R) was introduced was evaluated. As controls, strain NEIS-1804 (wild-type strain), a CYME CMQ453C (without the mutation)-introduced strain and a recombinant strain example were similarly evaluated. As the recombinant strain example, one obtained by introducing the gene of CYME_CMS462C (Bifunctional dihydrofolate reductase-thymidylate synthase) into strain NEIS-1804 was used.

To 25-cm² cell culture flasks (vent cap), 20 mL of MA2 medium was added, and algal cells were inoculated. The cells were photoautotrophically cultured at 40°C at 130 rpm for approximately a week. To the medium for the transformed strains, 200 µg/mL of chloramphenicol was added. Next, the culture solutions were subcultured to flasks containing 20 mL of 3% glycerol-containing MA2 medium (MA2 + 3% glycerol medium) to OD₇₅₀=0.5. The cells were mixotrophically cultured at 40°C at 130 rpm with application of light for three to five days. Next, the culture solutions were subcultured to flasks containing 20 mL of fresh MA2 + 3% glycerol medium to OD₇₅₀=0.5. The cells were heterotrophically cultured at 40°C at 130 rpm in the dark. About every week, 0.5 mL of the culture solutions were taken, and the OD₇₅₀ values were measured. The culture solutions were subcultured to flasks containing 20 mL of MA2 + 3% glycerol medium to OD₇₅₀=0.5 three weeks after starting the cultivation, and the cultivation was continued.

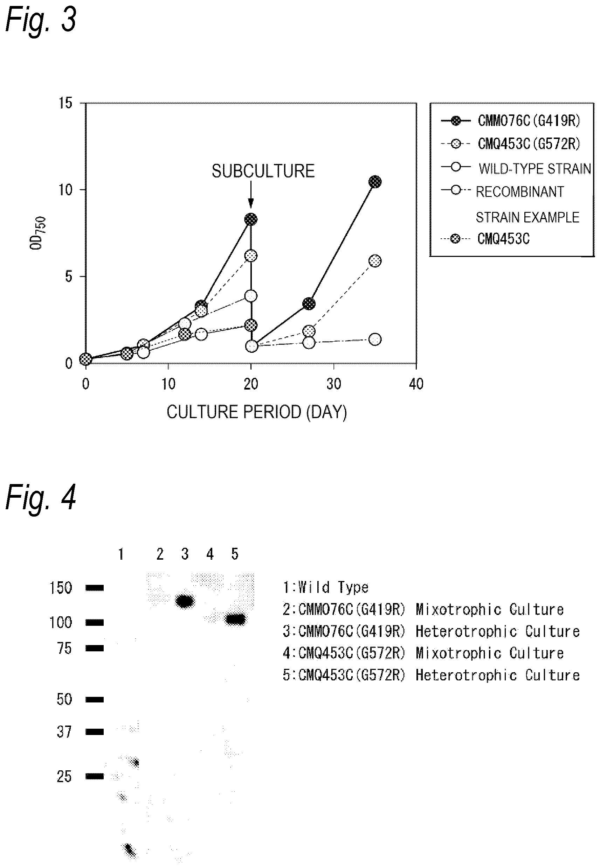

The results are shown in Fig. 3. The heterotrophic growth in the dark of the wild-type strain and the CYME_CMQ453C-introduced strain was slow. The heterotrophic growth rate of the recombinant strain example slightly increased compared to that of the wild-type strain, but the recombinant strain example hardly grew after the subculture. On the other hand, the heterotrophic growth rates of the CMM076C (G419R)-introduced strain and the CMQ453C (G572R)-introduced strain increased significantly compared to that of the wild-type strain. The maximum reached algal densities also increased significantly. The heterotrophic growth rates of the CMM076C (G419R)-introduced strain and the CMQ453C (G572R)-introduced strain remained high also after the subculture.

### <Western Blot Analysis>

Through Western Blot analysis, the protein expression of the introduced genes in the CMM076C (G419R)-introduced strain and the CMQ453C (G572R)-introduced strain was observed.

To 25-cm² cell culture flasks (vent cap), 20 mL of MA2 + 3% glycerol medium was added, and the algal cells were inoculated. The cells were cultured mixotrophically (with application of light) or heterotrophically (in the dark) at 40°C at 130 rpm. At the point where the algal density became OD₇₅₀ ~0.5, 1 mL of the culture solutions were taken and centrifuged for five minutes at room temperature at 4000 xg, and the supernatants were removed. The precipitated algal cells were suspended in 10 µL of MilliQ, and subsequently, 2 µL of SDS-PAGE Sample Loading Buffer (Takara Bio Inc., 786-701) to which β-mercaptoethanol was added at a certain concentration was added, followed by heating at 95°C for five minutes. The samples after heating were used as SDS-PAGE samples.

The entire amounts of the SDS-PAGE samples were applied to a precast gel Supercep Ace 5-20% (Wako, 194-15021) and were caused to migrate at 150 CV for 90 minutes. As the molecular marker, Precision Plus Dual Color Standard (Bio-Rad, #1610374) was used. A TransBlot Turbo^{™} mini PVDF transfer pack (Bio-rad, #1704156) was opened, and the Bottom side was placed on the stage of PoweredBlot Ace (WSE-4115, Atto). The gel after the completion of electrophoresis was cut into an appropriate size and placed on the PVDF membrane on the stage, and the Top side of the transfer pack was placed on the gel. After removing the air bubbles with a roller, the cover of PoweredBlot Ace was closed, and the gel was transferred at 25 CV (Rapid) for 10 minutes. The PVDF membrane was cut into an appropriate size and placed in a plastic container, and after adding 5 mL of TBS-T to the container, the container was shaken at room temperature for five minutes. The TBS-T was discarded, and 5 mL of PVDF Blocking Reagent (TOYOBO, NYPBR01) was added to the container. The container was shaken at room temperature for an hour. Next, the PVDF Blocking Reagent was discarded, and the PVDF membrane was rinsed with 5 mL of TBS-T, and 5 mL of TBS-T was added to the container. The container was shaken at room temperature for 15 minutes. Changing of TBS-T in the container and shaking at room temperature for five minutes were repeated twice.

Anti HA antibody-HRP conjugate (Wako, 011-21911) was diluted 2000-fold with Can Get Signal (registered trademark) Solution 1 (TOYOBO, NKB-201), and thus an antibody solution was prepared. The TBS-T in the container was discarded, and 10 mL of the antibody solution was added to the container. The container was shaken at room temperature for an hour to react the antibody. The antibody solution was discarded, and 5 mL of TBS-T was added. The container was shaken at room temperature for 10 minutes. Changing of TBS-T and shaking at room temperature for 10 minutes were repeated twice. Necessary amounts of Solution A and Solution B of ImmunoStar (registered trademark) Zeta (Wako, 297-72403) were mixed at 1:1. The mixed ImmunoStar Zeta was dropped on plastic wrap, and the PVDF membrane was dipped in such a manner that the transferred side faced downwards. While paying attention not to include any air bubbles between the PVDF membrane and the liquid, the PVDF membrane was left still for five minutes. Next, the PVDF membrane was lifted, and excess water was removed by absorbing with paper from the edge of the PVDF membrane. Then, with ChemiDoc XRS Plus Image Lab laptop system (Bio-rad, 1708265J1NPC) in the Chemi mode, the chemiluminescence of the PVDF membrane was detected, and pictures were taken.

The results are shown in Fig. 4. A band was observed at around 133 kDa with the CMM076C (G419R)-introduced strain, and the expression of CMM076C (G419R) was observed. A band was observed at around 117 kDa with the CMQ453C (G572R)-introduced strain, and the expression of CMQ453C (G572R) was observed. Both in the CMM076C (G419R)-introduced strain and the CMQ453C (G572R)-introduced strain, the signals of the introduced gene proteins in mixotrophic cultivation were weaker than the signals of the introduced gene proteins in heterotrophic cultivation. This is believed to be because other genes were expressed actively in mixotrophic cultivation and because the expression level of the introduced gene became relatively low compared to the entire gene expression level.

### <Search of CRY Gene of Haematococcuspluvialis>

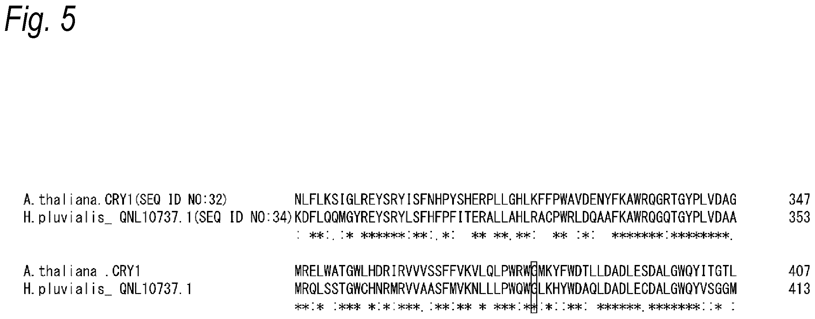

CRY gene of *Haematococcus pluvialis* (also called *Haematococcus lacustris*)*,* which is an alga of *Haematococcus* of Haematococcaceae of Volvocales, has already been identified (GenBank: QNL10737.1, W. Hang et al., Protein Expression and Purification, 2020 Aug;172:105633.). As a result of protein domain search in the Pfam database (EMBL-EBI, UK), CRY gene of *H. pluviali* (QNL10737.1; SEQ ID NOs: 33 and 34) had the FAD-binding domain. In an alignment with *Arabidopsis thaliana* CRY1 using BLAST search, the amino acid residues corresponding to D359 and V363 of CRY1 were not histidine in QNL10737.1. The amino acid residue (G386) corresponding to G380 of CRY1 was conserved and remained to be the glycine residue in QNL10737.1 (Fig. 5). In QNL10737.1, the amino acid sequence of the formula (1) was conserved (LLLPWQWGLKHY: SEQ ID NO: 35). By introducing a mutation into G386 of QNL10737.1, a dark active CRY can be obtained.

### <Search of CRY Genes of Nannochloropsis gaditana>

CRY genes were searched in *Nannochloropsis gaditana* (current *Microchloropsis gaditana*)*,* which is an alga of *Nannochloropsis* of Eustigmataceae of Eustigmatales.

Through protein-protein BLAST search of Database: non-redundant protein sequences, Organism: *Nannochloropsis gaditana* (taxid: 72520) using the amino acid sequence of *Arabidopsis thaliana* CRY1 as a query, three CRY candidate genes (EWM28467, EWM23893 and XP_005852637) were obtained. No homology with CRY1 was found in the C-terminal region in all the obtained CRY candidate genes. Percent identity (sequence identities) between the obtained CRY candidate genes and CRY1 were 23.1% to 34.0%.

Next, a phylogenetic analysis was conducted among the obtained CRY candidate gene group, *Arabidopsis thaliana* CRY1 and CYME_CMA044C (CRY-DASH). EWM28467 was closely related to CRY-DASH and was not CRY. On the other hand, the amino acid sequences of EWM23893 and XP_005852637 were similar, and both had a motif sequence of DNA photolyase. In Ochrophyta to which *Nannochloropsis* belongs, a case which has DNA photolyase activity but is still CRY is reported (S Coesel et al., EMBO Rep. 2009 Jun;10(6):655-61.). Therefore, it was believed that either or both of EWM23893 and XP_005852637 were CRY.

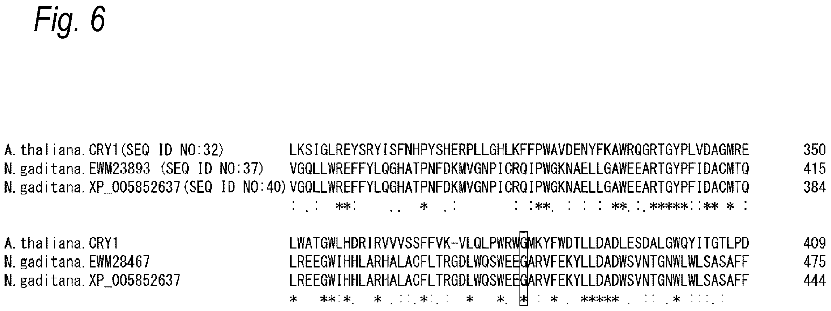

EWM23893 (SEQ ID NOs: 36 and 37) had the FAD-binding domain as a result of protein domain search in the Pfam database (EMBL-EBI, UK). In EWM23893, the amino acid residue (G446) corresponding to G380 of CRY1 was conserved and remained to be the glycine residue (Fig. 6). In EWM23893, the amino acid sequence of the formula (1) was conserved (LWQSWEEGARVF: SEQ ID NO: 38). By introducing a mutation to G446 of EWM23893, a dark active CRY can be obtained.

XP_005852637 (SEQ ID NOs: 39 and 40) had the FAD-binding domain as a result of protein domain search in the Pfam database (EMBL-EBI, UK). In XP_005852637, the amino acid residue (G415) corresponding to G380 of CRY1 was conserved and remained to be the glycine residue (Fig. 6). In XP_005852637, the amino acid sequence of the formula (1) was conserved (LWQSWEEGARVF: SEQ ID NO: 41). By introducing a mutation to G415, which is the amino acid corresponding to G380 of CRY1 gene, a dark active CRY can be obtained.

### <Search of CRY Genes of Phaeodactylum tricornutum>

CRY genes were searched in *Phaeodactylum tricornutum,* which is an alga of *Phaeodactylum* of Phaeodactylaceae of Naviculales.

Through protein-protein BLAST search of Database: non-redundant protein sequences, Organism: *Phaeodactylum tricornutum* strain CCAP 1055/1 (taxid: 556484) using the amino acid sequence *of Arabidopsis thaliana* CRY1 gene as a query, CRY candidate genes were obtained. No homology with CRY1 was found in the C-terminal region in all the obtained CRY candidate genes. Percent identity (sequence identities) between the obtained CRY candidate genes and CRY1 were 24.1% to 39.9%.

Next, a phylogenetic analysis was conducted among the obtained CRY candidate gene group, *Arabidopsis thaliana* CRY1 and CYME_CMA044C (CRY-DASH). Of the CRY candidate gene group, XP_002179379 was the most closely related to *Arabidopsis thaliana* CRY1. It is reported that XP_002179379 is close to CRY but not to CRY-DASH (M. Juhas et al., FEBS.J.(2014)). Therefore, it was concluded that XP_002179379 is a CRY gene.

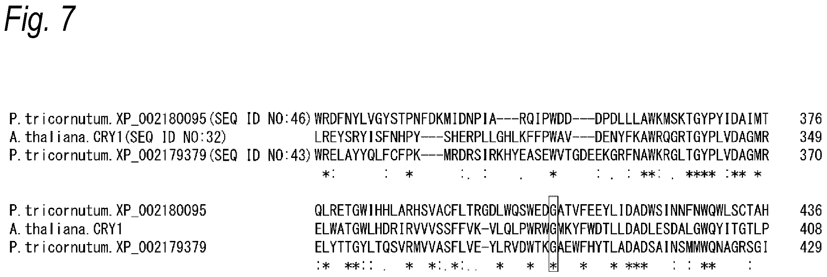

XP_002179379 (SEQ ID NOs: 42 and 43) had the FAD-binding domain as a result of protein domain search in the Pfam database (EMBL-EBI, UK). In an alignment with *Arabidopsis thaliana* CRY1 using BLAST search, the amino acid residues of XP_002179379 corresponding to D359 and V363 of CRY1 were not histidine. In XP_002179379, the amino acid residue (G401) corresponding to G380 of CRY1 was conserved and remained to be the glycine residue (Fig. 7). In XP_002179379, the amino acid sequence of the formula (1) was conserved (LRVDWTKGAEWF: SEQ ID NO: 44). By introducing a mutation to G401 of XP_005852637, a dark active CRY can be obtained.

As a result of the phylogenetic analysis, XP_002180095 (SEQ ID NOs: 45 and 46) was slightly distantly related to *Arabidopsis thaliana* CRY1. However, XP_002180095 has been reported to have DNA photolyase activity as PtCPF1 but is still an animal-like cryptochrome (S Coesel et al., EMBO Rep. 2009 Jun;10(6):655-61.). XP_002180095 had the FAD-binding domain as a result of protein domain search in the Pfam database (EMBL-EBI, UK). As a result of an alignment with *Arabidopsis thaliana* CRY1 using BLAST search, the amino acid residues of XP_002180095 corresponding to D359 and V363 of CRY1 were histidine. In XP_002180095, the amino acid residue (G408) corresponding to G380 of CRY1 was conserved and remained to be the glycine residue (Fig. 7). In XP_002180095, the amino acid sequence of the formula (1) was conserved (LWQSWEDGATVF: SEQ ID NO: 47). By introducing a mutation to G408 of XP_002180095, a dark active CRY can be obtained.

### <Search of CRY Genes of Diacronema lutheri>

CRY genes were searched in *Diacronema lutheri* (also called *Pavlova lutheri*)*,* which is an alga of *Diacronema* of Pavlovaceae of Pavlovales.

Through protein-protein BLAST search of Database: non-redundant protein sequences, Organism: *Diacronema* (*Pavlova*) *lutheri* (taxid: 2081491) using the amino acid sequence *of Arabidopsis thaliana* CRY1 as a query, CRY candidates were obtained. No homology with CRY1 could be found in the C-terminal region in all the obtained CRY candidates. Percent identity (sequence identities) between the obtained CRY candidate genes and CRY1 were 26.9% to 36.7%.

Next, a phylogenetic analysis was conducted among the obtained CRY candidate gene group, *Arabidopsis thaliana* CRY1 and CYME_CMA044C (CRY-DASH). KAG8467638 was closely related to CRY-DASH and was not CRY. Of the CRY candidate gene group, KAG8458188 was the most closely related to *Arabidopsis thaliana* CRY1. KAG8463402 was not closely related to *Arabidopsis thaliana* CRY1 and CRY-DASH.

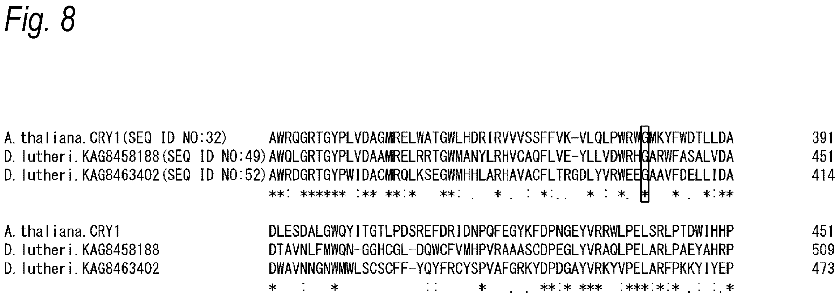

KAG8458188 (SEQ ID NOs: 48 and 49) had the FAD-binding domain as a result of protein domain search in the Pfam database (EMBL-EBI, UK). In an alignment with *Arabidopsis thaliana* CRY1 using BLAST search, the amino acid residues of KAG8458188 corresponding to D359 and V363 of CRY1 were histidine, and thus KAG8458188 was believed to have DNA photolyase activity. In the His-His-Leu-Ala-Arg-His motif, which is important for the DNA photolyase activity, however, the second and the last histidine residues were conserved in KAG8458188, but the first histidine residue was mutated to an alanine residue. Thus, it was believed that KAG8458188 had partially or completely lost the DNA photolyase activity and that the primary function thereof was that of CRY. In KAG8458188, the amino acid residue (G440) corresponding to G380 of CRY1 was conserved and remained to be the glycine residue (Fig. 8). In KAG8458188, the amino acid sequence of the formula (1) was conserved (LLVDWRHGARWF: SEQ ID NO: 50). By introducing a mutation to G440 of KAG8458188, a dark active CRY can be obtained.

KAG8463402 (SEQ ID NOs: 51 and 52) had the FAD-binding domain as a result of protein domain search in the Pfam database (EMBL-EBI, UK). In an alignment with *Arabidopsis thaliana* CRY1 using BLAST search, the amino acid residues of KAG8463402 corresponding to D359 and V363 of CRY1 were histidine. In Ochrophyta to which *Diacronema* belongs, a case which has DNA photolyase activity but is still CRY is reported (S Coesel et al., EMBO Rep. 2009 Jun;10(6):655-61.). Therefore, KAG8463402 was also believed to have the function of CRY. In KAG8463402, the amino acid residue (G403) corresponding to G380 of CRY1 was conserved and remained to be the glycine residue (Fig. 8). In KAG8463402, the amino acid sequence of the formula (1) was conserved (LYVRWEEGAAVF: SEQ ID NO: 53). By introducing a mutation to G403 of KAG8463402, a dark active CRY can be obtained.

### <Search of CRY Genes of Chaetoceros muelleri>

CRY genes were searched in *Chaetoceros muelleri,* which is an alga of *Chaetoceros* of Chaetocerotaceae of Chaetocerotales.

Through nucleotide-protein BLAST search of Database: ASM1969354v1 GenBank assembly [GCA_019693545.1] and Organism: *Chaetoceros muelleri* (taxid: 265525) using the amino acid sequence *of Arabidopsis thaliana* CRY1 as a query, CRY candidates were obtained. No homology with CRY1 could be found in the C-terminal region in all the obtained CRY candidates. Percent identity (sequence identities) between the obtained CRY candidate genes and CRY1 were 24.2% to 38.2%.

Next, a phylogenetic analysis was conducted among the obtained CRY candidate gene group, *Arabidopsis thaliana* CRY1 and CYME_CMA044C (CRY-DASH). Of the CRY candidate gene group, GENE1 was the most closely related to *Arabidopsis thaliana* CRY1. GENE2 and GENE3 were not closely related to *Arabidopsis thaliana* CRY1 and CRY-DASH.

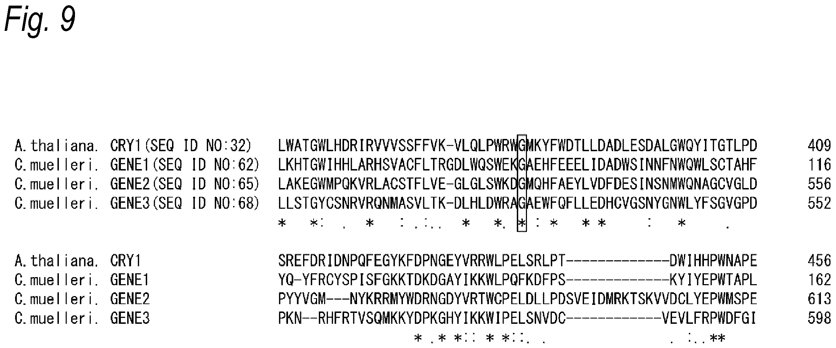

GENE1 (SEQ ID NOs: 61 and 62) had the FAD-binding domain as a result of protein domain search in the Pfam database (EMBL-EBI, UK). In an alignment with *Arabidopsis thaliana* CRY1 using BLAST search, the amino acid residues of GENE1 corresponding to D359 and V363 of CRY1 were histidine. In Ochrophyta to which *Chaetoceros* belongs, a case which has DNA photolyase activity but is still CRY is reported (S Coesel et al., EMBO Rep. 2009 Jun;10(6):655-61.). Therefore, GENE1 was also believed to have the function of CRY. In GENE1, the amino acid residue (G87) corresponding to G380 of CRY1 was conserved and remained to be the glycine residue (Fig. 9). In GENE1, the amino acid sequence of the formula (1) was conserved (LWQSWEKGAEHF: SEQ ID NO: 63). By introducing a mutation to G87 of GENE1, a dark active CRY can be obtained.

GENE2 (SEQ ID NOs: 64 and 65) had the FAD-binding domain as a result of protein domain search in the Pfam database (EMBL-EBI, UK). In GENE2, the amino acid residue (G527) corresponding to G380 of CRY1 was conserved and remained to be the glycine residue (Fig. 9). In GENE2, the amino acid sequence of the formula (1) was conserved (LGLSWKDGMQHF: SEQ ID NO: 66). By introducing a mutation to G527, which is the amino acid residue corresponding to G380 of CRY1 gene, a dark active CRY can be obtained.

GENE3 (SEQ ID NOs: 67 and 68) had the FAD-binding domain as a result of protein domain search in the Pfam database (EMBL-EBI, UK). In GENE3, the amino acid residue (G523) corresponding to G380 of CRY1 was conserved and remained to be the glycine residue (Fig. 9). In GENE3, the amino acid sequence of the formula (1) was conserved (LHLDWRAGAEWF: SEQ ID NO: 69). By introducing a mutation to G523, which is the amino acid residue corresponding to G380 of CRY1 gene, a dark active CRY can be obtained.

### <Search of CRY Genes of Chaetoceros gracilis>

CRY genes were searched in *Chaetoceros gracilis,* which is an alga of *Chaetoceros* of Chaetocerotaceae of Chaetocerotales.

Through nucleotide-protein BLAST search of Database: PRJDB12186 and Organism: *Chaetoceros gracilis* (taxid: 184592) using the amino acid sequence of *Arabidopsis thaliana* CRY1 as a query, CRY candidates were obtained. No homology with CRY1 could be found in the C-terminal region in all the obtained CRY candidates. Percent identity (sequence identities) between the obtained CRY candidate genes and CRY1 were 24.1% to 34.2%.

Next, a phylogenetic analysis was conducted among the obtained CRY candidate gene group, *Arabidopsis thaliana* CRY1 and CYME_CMA044C (CRY-DASH). GENE4 was closely related to CRY-DASH and was not CRY. Of the CRY candidate gene group, GENE5 was the most closely related to *Arabidopsis thaliana* CRY1. GENE6 and GENE7 were not closely related to *Arabidopsis thaliana* CRY1 and CRY-DASH.

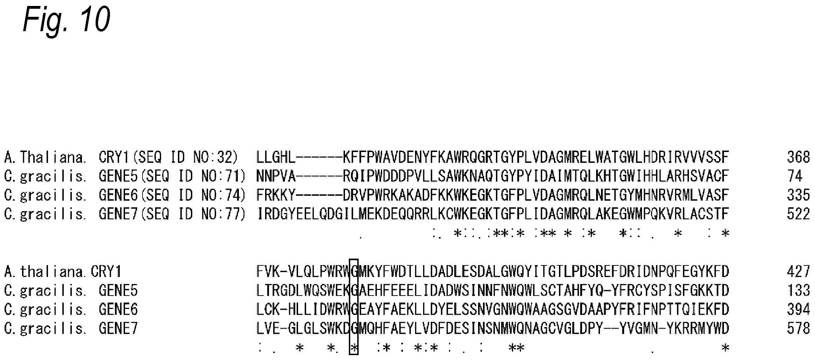

GENE5 (SEQ ID NOs: 70 and 71) had the FAD-binding domain as a result of protein domain search in the Pfam database (EMBL-EBI, UK). In an alignment with *Arabidopsis thaliana* CRY1 using BLAST search, the amino acid residues of GENE5 corresponding to D359 and V363 of CRY1 were histidine. In Ochrophyta to which *Chaetoceros* belongs, a case which has DNA photolyase activity but is still CRY is reported (S Coesel et al., EMBO Rep. 2009 Jun;10(6):655-61.). Therefore, GENE5 was also believed to have the function of CRY. In GENE5, the amino acid residue (G87) corresponding to G380 of CRY1 was conserved and remained to be the glycine residue (Fig. 10). In GENE5, the amino acid sequence of the formula (1) was conserved (LWQSWEKGAEHF: SEQ ID NO: 72). By introducing a mutation to G87 of GENE5, a dark active CRY can be obtained.

GENE6 (SEQ ID NOs: 73 and 74) had the FAD-binding domain as a result of protein domain search in the Pfam database (EMBL-EBI, UK). In GENE6, the amino acid residue (G347) corresponding to G380 of CRY1 was conserved and remained to be the glycine residue (Fig. 10). In GENE6, the amino acid sequence of the formula (1) was conserved (LLIDWRWGEAYF: SEQ ID NO: 75). By introducing a mutation to G347, which is the amino acid residue corresponding to G380 of CRY1 gene, a dark active CRY can be obtained.

GENE7 (SEQ ID NOs: 76 and 77) had the FAD-binding domain as a result of protein domain search in the Pfam database (EMBL-EBI, UK). In GENE7, the amino acid residue (G534) corresponding to G380 of CRY1 was conserved and remained to be the glycine residue (Fig. 10). In GENE7, the amino acid sequence of the formula (1) was conserved (LGLSWKDGMQHF: SEQ ID NO: 78). By introducing a mutation to G534, which is the amino acid residue corresponding to G380 of CRY1 gene, a dark active CRY can be obtained.

Preferable Examples of the invention have been explained above, but the invention is not limited to the Examples. Addition, omission, replacement and other change of the components can be made in the scope which does not depart from the gist of the invention. The invention is not limited by the explanations given above but is limited only by the scope of the attached claims.

## Claims

1. An alga having a dark active cryptochrome whose activation is maintained in the dark.

2. The alga according to claim 1 which has a higher growth rate in the dark than that of an alga of the same species without the dark active cryptochrome.

3. The alga according to claim 1 which has a higher maximum reached algal density in the dark than that of an alga of the same species without the dark active cryptochrome.

4. The alga according to claim 1, wherein the dark active cryptochrome has an amino acid sequence having a mutation in a flavin adenine dinucleotide-binding domain compared to the amino acid sequence of a dark inactive cryptochrome which is inactivated in the dark.

5. The alga according to claim 4, wherein the flavin adenine dinucleotide-binding domain of the dark inactive cryptochrome comprises an amino acid sequence represented by the following formula (1),
LXZXWXXGXXXJ (1)
[wherein in the formula, L represents a leucine residue, W represents a tryptophan residue, G represents a glycine residue, Z represents a hydrophobic amino acid residue or a polar uncharged amino acid residue, J represents a hydrophobic amino acid residue, and X represents any amino acid residue.]

6. The alga according to claim 5, wherein the dark active cryptochrome has an amino acid sequence having a mutation in the amino acid sequence represented by the formula (1) compared to the amino acid sequence of the dark inactive cryptochrome.

7. The alga according to claim 6, wherein the dark active cryptochrome comprises an amino acid sequence in which the glycine residue at position 8 of the amino acid sequence represented by the formula (1) has been substituted with a basic amino acid residue.

8. The alga according to any one of claims 1 to 7, wherein the alga is a unicellular alga.

9. A method for improving growth of an alga in the dark, comprising introducing a polynucleotide encoding a dark active cryptochrome into an alga.

10. A method for producing an alga with improved growth in the dark, comprising introducing a polynucleotide encoding a dark active cryptochrome into an alga.

11. A dark active cryptochrome whose activation is maintained in the dark, having an amino acid sequence selected from the group consisting of (a) to (c) below:
(a) an amino acid sequence in which the glycine residue at position 8 of an amino acid sequence represented by the following formula (1) has been substituted with a basic amino acid residue in the amino acid sequence of SEQ ID NO: 12, 14, 34, 37, 40, 43, 46, 49, 52, 62, 65, 68, 71, 74 or 77,
LXZXWXXGXXXJ (1)
[wherein in the formula, L represents a leucine residue, W represents a tryptophan residue, G represents a glycine residue, Z represents a hydrophobic amino acid residue or a polar uncharged amino acid residue, J represents a hydrophobic amino acid residue, and X represents any amino acid residue];
(b) an amino acid sequence having a mutation of one amino acid or a plurality of amino acids in the amino acid sequence of SEQ ID NO: 12, 14, 34, 37, 40, 43, 46, 49, 52, 62, 65, 68, 71, 74 or 77, wherein the mutation comprises a mutation of substituting the glycine residue at position 8 of the amino acid sequence represented by the formula (1) with a basic amino acid residue; and
(c) an amino acid sequence which has a sequence identity of 70% or more with the amino acid sequence of SEQ ID NO: 12, 14, 34, 37, 40, 43, 46, 49, 52, 62, 65, 68, 71, 74 or 77 and in which the glycine residue at position 8 of the amino acid sequence represented by the formula (1) has been substituted with a basic amino acid residue.

12. A polynucleotide encoding the dark active cryptochrome according to claim 11.
